# EUROPEAN PATENT APPLICATION

(11) **EP 2 394 649 A1**
(43) Date of publication of application: **14.12.2011**
(21) Application number: 10738198.0
(22) Date of filing: 04.02.2010
(51) Int. Cl.: A61K 31/44, A61P 35/00, C08B 37/16, C07D 213/75

(54) **PHARMACEUTICAL COMPOSITIONS CONTAINING PYRIDYL CYANOGUANIDINES, PREPARATION METHODS AND USES THEREOF**

(30) Priority: 06.02.2009 CN 200910067820; 04.02.2010 CN 201010105331
(71) Applicant: Tianjin Hemay Bio-Tech Co., Ltd., Tianjin 300457 (CN); Tianjin Michele Sci-Tech Development Co., Ltd., Tianjin 300192 (CN)
(72) Inventor: ZHANG, Hesheng, Tianjin 300457 (CN); ZHANG, Qinghua, Tianjin 300457 (CN); CHEN, Yingwei, Tianjin 300457 (CN); HUO, Aihong, Tianjin 300457 (CN)
(74) Representative: Swinkels, Bart Willem
(86) International application number: PCT/CN2010/000156
(87) International publication number: WO 2010/088842

(57) **Abstract**

The compositions formed from pyridyl cyanoguanidines and cyclodextrins, cyclodextrin derivatives and/or surfactants with solubilization, preparation methods and uses thereof.

## Description

### Technical Field

The invention relates to a pharmaceutical composition comprising pyridyl cyanoguanidine, process for preparing the same and use thereof.

### Technical Background

It was initially found that pyridyl cyanoguanidine such as pinadicil (N-1,2,2-trimethylpropyl-N'-cyano-N"-(4-pyridyl)guanidine) is a potassium channel opener and therefore pyridyl cyanoguanidine was developed as a antihypertensive agent. If the side chain of pinadicil was replaced with longer aryl group-containing side chain, pyridyl cyanoguanidine will lose the antihypertensive activity. However, in another aspect, it was found that such pyridyl cyanoguanidine has the antitumor activity when it is adminstered orally in a rat model with Yoshida ascitic tumor.

Various kinds of pyridyl cyanoguanidines with antiproliferative activities are disclosed in such as EP 660823, WO 98/54141, WO 98/54143, WO 98/54144, WO 98/54145, WO 00/61559 and WO 00/61561, WO 98/54146, WO 2002/094813, US 5696140. The structure-activity relation (SAR) of such compounds is discussed in C. Schou et al., Bioorganic and Medicinal Chemistry Letters 7(24), 1997, 3095-3100, wherein the antiproliferative effects of a large number of pyridyl cyanoguanidines on various human lung cancer and breast cancer cell lines as well as normal human fibroblasts have been tested *in vitro. In vivo* tests for the compounds have also been carried out using nude mice with human lung cancer tumor xenotransplantation. A specific compound (N-(6-(4-chlorophenoxy)hexyl)-N'-cyano-N"-(4-pyridyl)guanidine) with high *in vitro* antiproliferative activity and high antitumor activity in nude mice model was selected according to the SAR analysis.

Further test results of *in vitro* and *in vivo* tests of the compound N-(6-(4-chlorophenoxy)hexyl)-N'-cyano-N"-(4-pyridyl)guanidine are reported in P-J V Hjarnaa et al., Cancer Res. 59, 1999, 5751-5757. The compound exhibits comparative *in vitro* efficacy with cytostatic agents daunorubicin and paclitaxel as control, and also shows significantly lower antiproliferative activity to normal human endothelial cells. In the *in vivo* test carried out with nude mice transplanted human tomour cells, the compound shows potent antitumour activity and can also oppose tumor cells with drug-tolerance to conventional antitumor drugs such as paclitaxel.

Although as stated above pyridyl cyanoguanidine is a great promising antitumor medicament and has the quite notable antitumor activity, pyridyl cyanoguanidine has high lipotropy and therefore is an insoluble compound. CN 01819244.0 discloses that the solubility of N-(6-(4-chlorophenoxy)-hexyl)-N'-cyano-N"-(4-pyridyl)-guanidine in water is 0.0002 mg/ml, which means that the compound generally can only be administered orally. However, there exists a big issue as to the compliance of patients when the drugs are administered orally because many patients suffering from cancers are very weak. Therefore, a conventional way to increase the solubility of an insoluble compound is to prepare a water-soluble prodrug. Prodrugs of pyridyl cyanoguanidine are disclosed in WO 2003/097602, WO 2003/097601 and WO 2002/042265.

Since β-cyclodextrin (β-CD) was obtained successfully from fermentation liquor by S. Dentin firstly in 1903, a great progress in the preparation, property and application of cyclodextrins has been achieved by researchers. Therefore, β-cyclodextrin has been used in a variety of fields. The pharmaceutical application of cyclodextrins and derivatives thereof cause great interests of researchers. Pharmaceutical cyclodextrin clathrates increasingly exhibit unique properties and application values in improving solubility, dissolution rate and bioavailability of a medicament. β-CD has been recorded as oral excipients in pharmacopoeia of the United States, Japan and China, *etc.*

### Discription of the Invention

The invention discloses a composition comprising at least one of compounds of formula (I) and a cyclodextrin, a cyclodextrin derivative and/or other surfactants with solubilization, process for preparing the same and use thereof, 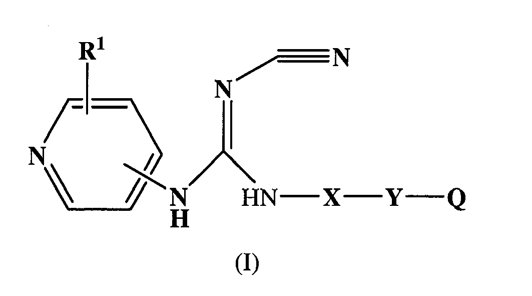 wherein
the connecting position with the pyridine ring is at the 3- or 4-position of the pyridine ring;
R¹ represents 0 to 4 following identical or different substituents: F, Cl, Br, NO₂, OH, COOH, CF₃, NR³R⁴, OR³, COOC₁₋₄ alkyl, C₁₋₄ alkyl or CN;
X represents C₄₋₂₀ alkylene;
Y represents a covalent bond, O, S, S(O), S(O)₂, NR³, -OC(O)-, -C(O)O-, -NHC(O)- or -C(O)NH-;
Q represents -C(O)OC₁₋₆ alkyl, a five- or six-membered aromatic heterocyclic ring containing 1 to 2 nitrogen atoms, a compound of formula (II) or a compound of formula (III), wherein the dashed lines in the compound of formula (II) and the compound of formula (III) represent the connecting position of chemical bonds, and R¹ in the compound of formula (III) is defined as R¹ in the compound of formula (I),

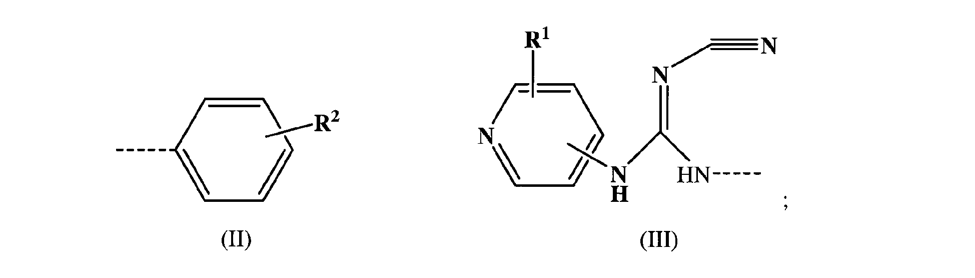

R² represents 0 to 5 following identical or different substituents: F, Cl, Br, NO₂ OH, COOH, CF₃, NR³R⁴, OR³, COOC₁₋₄ alkyl, C₁-₄ alkyl, SC₁₋₄ alkyl, S(O)C₁₋₄ alkyl, S(O)₂C₁₋₄ alkyl or CN.

Unless otherwise specified, R³ and R⁴ in the invention each represent H, C₁₋₄ alkyl, C(O)C₁₋₄ alkyl or S(O)₂C₁₋₄ alkyl, respectively.

C₁₋₄ alkyl in the invention represents a branched or linear, saturated or unsaturated monovalent alkyl containing 1 to 4 carbon atoms, which can be substituted with 0 to 3 following substituents: OH, F, Cl, NO₂ CN, SH, NHC(O)NH₂, OR³, SR³, COOH, C(O)NR³R⁴, NR³R⁴, a phenyl or a substituted phenyl, wherein the substituted phenyl refers to a phenyl containing 1 to 4 following identical or different groups: OH, F, Cl, NO₂ CN, SH, NHC(O)NH₂, OR³, SR³, R³, COOH, C(O)NR³R⁴ or NR³R⁴.

C₁₋₆ alkyl in the invention represents a branched or linear, saturated or unsaturated monovalent alkyl containing 1 to 6 carbon atoms, which can be substituted with 0 to 3 following groups: OH, F, Cl, NO₂ CN, SH, NHC(O)NH₂, OR³, SR³, COOH, C(O)NR³R⁴, NR³R⁴, a phenyl or a substituted phenyl, wherein the substituted phenyl refers to a phenyl containing 1 to 4 following identical or different groups: OH, F, Cl, NO₂, CN, SH, NHC(O)NH₂, OR³, SR³, R³, COOH, C(O)NR³R⁴ or NR³R⁴.

C₄₋₂₀ alkylene in the invention represents a branched or linear, saturated or unsaturated divalent alkyl containing 4 to 20 carbon atoms, which can be substituted with 0 to 6 following identical or different groups: OH, F, Cl, NO₂, CN, SH, NHC(O)NH₂, OR³, SR³, COOH, C(O)NR³R⁴, NR³R⁴, a phenyl or a substituted phenyl, wherein the substituted phenyl refers to a phenyl containing 1 to 4 following identical or different groups: OH, F, Cl, NO₂ CN, SH, NHC(O)NH₂, OR³, SR³, R³, COOH, C(O)NR³R⁴ or NR³R⁴.

The five- or six-membered aromatic heterocyclic ring containing 1 to 2 nitrogen atoms in the invention represents pyrrolyl, pyridyl or pyrimidinyl, which can be substituted with 0 to 4 following identical same or different groups: OH, F, Cl, NO₂, CN, SH, NHC(O)NH₂, OR³, SR³, COOH, C(O)NR³R⁴ or NR³R⁴.

The composition comprising a compound of formula (I) provided in the invention significantly increase the solubility of the compound of formula (I) in an aqueous solution and therefore it gives more options for clinical practice of the compound of formula (I).

In the composition comprising at least one of a compound of formula (I) in the invention, the compound of formula (I) refers to a compound of formula (I), a pharmaceutically acceptable salt of a compound of formula (I), a solvate of a compound of formula (I), a solvate of a pharmaceutically acceptable salt of a compound of formula (I), a tautomer of a compound of formula (I), a pharmaceutically acceptable salt of a tautomer of a compound of formula (I), a solvate of a tautomer of a compound of formula (I) or a solvate of a pharmaceutically acceptable salt of a tautomer of a compound of formula (I).

A tautomer of a compound of formula (I) as described in the invention includes, but is not limited to the structure of a compound of formula (IV): 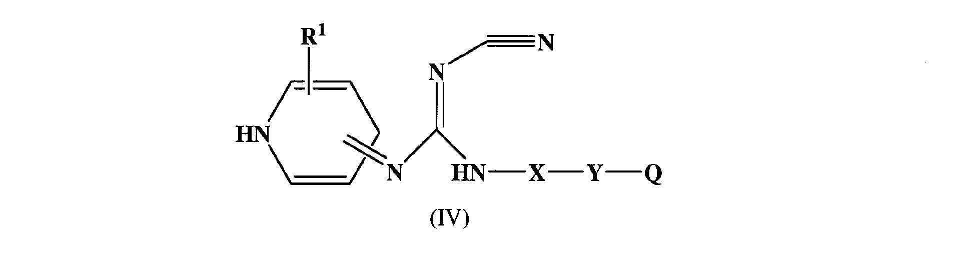 wherein
the connecting position with the pyridine ring is at the 3- or 4-position of the pyridine ring;
R¹ represents 0 to 4 following identical or different substituents: F, Cl, Br, NO₂ OH, COOH, CF₃, NR³R⁴, OR³, COOC₁₋₄ alkyl, C₁₋₄ alkyl or CN;
X represents C₄₋₂₀ alkylene;
Y represents a covalent bond, O, S, S(O), S(O)₂, NR³, -OC(O)-, -C(O)O-, -NHC(O)- or -C(O)NH-;
Q represents -C(O)OC₁₋₆ alkyl, a five- or six-membered aromatic heterocyclic ring containing 1 to 2 nitrogen atoms, a compound of formula (II) or a compound of formula (III), wherein the dashed lines in the compound of formula (II) and the compound of formula (III) represent the connecting position of chemical bonds, and R¹ in the compound of formula (III) is defined as R¹ in the compound of formula (I), 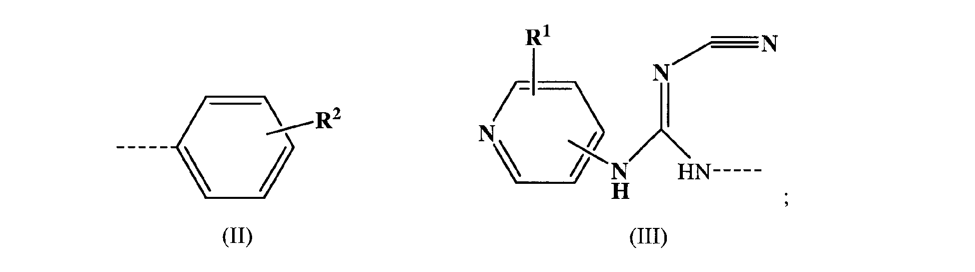
R² represents 0 to 5 following identical or different substituents: F, Cl, Br, NO₂ OH, COOH, CF₃, NR³R⁴, OR³, COOC₁₋₄ alkyl, C₁₋₄ alkyl, SC₁₋₄ alkyl, S(O)C₁₋₄ alkyl, S(O)₂C₁₋₄ alkyl or CN;
Unless otherwise specified, R³ and R⁴ in the invention each represent H, C₁₋₄ alkyl, C(O)C₁₋₄ alkyl or S(O)₂C₁₋₄ alkyl, respectively.

A composition provided in the invention is prepared with at least one of a compound of formula (I), a pharmaceutically acceptable salt of a compound of formula (I), a solvate of a compound of formula (I), a solvate of a pharmaceutically acceptable salt of a compound of formula (I), a tautomer of a compound of formula (I), a pharmaceutically acceptable salt of a tautomer of a compound of formula (I), a solvate of a tautomer of a compound of formula (I) or a solvate of a pharmaceutically acceptable salt of a tautomer of a compound of formula (I), and a cyclodextrin and/or a cyclodextrin derivative.

A composition provided in the invention is prepared with at least one of a compound of formula (I), a pharmaceutically acceptable salt of a compound of formula (I), a solvate of a compound of formula (I), a solvate of a pharmaceutically acceptable salt of a compound of formula (I), a tautomer of a compound of formula (I), a pharmaceutically acceptable salt of a tautomer of a compound of formula (I), a solvate of a tautomer of a compound of formula (I) or a solvate of a pharmaceutically acceptable salt of a tautomer of a compound of formula (I), and a surfactant with solubilization.

Another composition provided in the invention is prepared with at least one of a compound of formula (I), a pharmaceutically acceptable salt of a compound of formula (I), a solvate of a compound of formula (I), a solvate of a pharmaceutically acceptable salt of a compound of formula (I), a tautomer of a compound of formula (I), a pharmaceutically acceptable salt of a tautomer of a compound of formula (I), a solvate of a tautomer of a compound of formula (I) or a solvate of a pharmaceutically acceptable salt of a tautomer of a compound of formula (I), and a cyclodextrin and/or a cyclodextrin derivative and a surfactant with solubilization.

Where a composition provided in the invention comprises a cyclodextrin or a cyclodextrin derivative, the cyclodextrin or cyclodextrin derivative is selected one or more than one of the following: α-cyclodextrin, β-cyclodextrin (β-CD), γ-cyclodextrin, δ-cyclodextrin, (C₁₋₄ alkyl)-α-cyclodextrin, (C₁₋₄ alkyl)-β-cyclodextrin, (C₁₋₄ alkyl)-γ-cyclodextrin, (hydroxy-C₁₋₄ alkyl)-α-cyclodextrin, (hydroxy-C₁₋₄ alkyl)-β-cyclodextrin, (hydroxy-C₁₋₄ alkyl)-γ-cyclodextrin, (carboxy-C₁₋₄ alkyl)-α-cyclodextrin, (carboxy-C₁₋₄ alkyl)-β-cyclodextrin, (carboxy-C₁₋₄ alkyl)-γ-cyclodextrin, diethylaminoethyl-α-cyclodextrin, diethylaminoethyl-β-cyclodextrin, diethylaminoethyl-γ-cyclodextrin, dihydroxy (C₁₋₄ alkyl)-α-cyclodextrin, dihydroxy (C₁₋₄ alkyl)-β-cyclodextrin, dihydroxy (C₁₋₄ alkyl)-γ-cyclodextrin, sulfobutylether-α-cyclodextrin, sulfobutylether-β-cyclodextrin and sulfobutylether-γ-cyclodextrin.

The cyclodextrin or cyclodextrin derivative suitable for preparing a pharmaceutical composition comprising a compound of formula (I) is α-cyclodextrin, β-cyclodextrin, γ-cyclodextrin, hydroxyethyl-β-CD, hydroxypropyl-β-CD, dihydroxypropyl-β-CD, sulfobutylether-β-CD, methyl-β-CD, dimethyl-β-CD, randomly dimethylated-β-CD, randomly methylated-β-CD, carboxy methyl-β-CD, carboxy methylethyl-β-CD, diethyl-β-CD, tris-O-methyl-β-CD, tris-O-ethyl-β-CD, tris-O-butyryl-β-CD, tris-O-valeryl-β-CD, bis-O-caproyl-β-CD, glucosyl-β-CD, maltosyl-β-CD, maltotriosyl-β-CD, succinyl-dimethyl-β-CD, maleyl-dimethyl-β-CD, sulfopropyl-β-CD, 6-carboxymethyl-β-CD or hydroxypropyl-γ-cyclodextrin. The cyclodextrin or cyclodextrin derivative more suitable for preparing a pharmaceutical composition comprising a compound of formula (I) is α-cyclodextrin, β-cyclodextrin, γ-cyclodextrin, hydroxyethyl-β-CD, hydroxypropyl-β-CD, sulfobutylether-β-CD, methyl-β-CD, dimethyl-β-CD, carboxy methyl-β-CD, glucosyl-β-CD, maltosyl-β-CD, succinyl-dimethyl-β-CD, maleyl-dimethyl-β-CD, sulfopropyl-β-CD or hydroxypropyl-γ-cyclodextrin. The cyclodextrin or cyclodextrin derivative particularly suitable for preparing a pharmaceutical composition comprising a compound of formula (I) are: β-cyclodextrin, hydroxyethyl-β-CD, hydroxypropyl-β-CD, sulfobutylether-β-CD, glucosyl-β-CD, sulfopropyl-β-CD or hydroxypropyl-γ-cyclodextrin.

Where an α-cyclodextrin, β-cyclodextrin or γ-cyclodextrin in the invention is substituted, the substitution can be any substitution suitable for a pharmaceutical excipient, which may be a single substitution or a combination of different substitutions. For example, the substitution range of sulfobutylether-β-CD may be from 1 to 20. A sulfobutylether-β-CD having a substitution of 7 may be a sulfobutylether-β-CD having a single substitution of 7 or a mixture of sulfobutylether-β-CDs having different substitutions but a mean substitution of 7. The appropriate substitution range of sulfobutylether-β-CD suitable for preparing a composition with a compound of formula (I) is from 3 to 8, more appropriate substitution range of sulfobutylether-β-CD suitable for preparing a composition with a compound of formula (I) is from 5 to 8, particularly appropriate substitution range of sulfobutylether-β-CD suitable for preparing a composition with a compound of formula (I) is from 6 to 8, the most appropriate substitution range of sulfobutylether-β-CD suitable for preparing a composition with a compound of formula (I) is from 6 to 7.1. Where an α-cyclodextrin, β-cyclodextrin or γ-cyclodextrin in the invention is substituted, the substitution can be took place at any possible positions, and can be a combination of different positions. For example, a hydroxypropyl-β-CD can be substituted at the 2-position, *i.e.* 2-hydroxypropyl-β-CD; or at the 3-position, *i.e.* 3-hydroxypropyl-β-CD; or at 6-position, *i.e*. 6-hydroxypropyl-β-CD; or a mixture of hydroxypropyl-β-CDs substituted at the 2-position, 3-position and/or 6-position. Unless otherwise specified, sulfobutylether-β-CD and hydroxypropyl-β-CD used in the invention are a mixture.

Where a pharmaceutical compositions disclosed in the invention comprises a surfactant with solubilization, the surfactant with solubilization is selected from one or more than one of the following: Tween-20, Tween-80, Tween-40, polyethylene glycol, polyoxyethylene castor oil, polyoxyethylene hydrogenated castor oil, polyvinylpyrrolidone, poloxamer(poloxamer), lecithin, soy lecithin, cephalin, phosphatidic acid, dipalmitoylphosphatidylcholine, hydrophosphatidyl ethanolamines, phosphatidylserine, cholesterol, diamino cholesterol, soy sterylacylglucoside, soysterol or ergosterol.

Compounds of formula (I) suitable for preparing a composition as a pharmaceutically active component are those compounds, in which R¹ represents 0 to 4 identical or different F, Cl, OH, NO₂, CF₃, CH₃, CHF₂ or CH₂F. Compounds of formula (I) more suitable for preparing a composition as a pharmaceutically active components are those compounds, in which R¹ represents 0 to 2 identical or different F, Cl, OH, CH₃ or CF₃. Compounds of formula (I) particularly suitable for preparing a composition as a pharmaceutically active component are those compounds, in which R¹ represents 0 to 1 identical or different of F or OH.

Compounds of formula (I) suitable for preparing a composition as a pharmaceutically active component are those compounds, in which Q represents a compound of formula (II) and R² represents 0 to 5 following identical or different substituents: F, Cl, Br, NO₂, OH, COOH, CF₃, CHF₂, CH₂F, N(CH₃)₂, N(CH₂CH₃)₂, N(CH₃)(CH₂CH₃), CH₃, CH₂CH₃, CH₂CH₂CH₃, CH(CH₃)₂, OCH₃, OCH₂CH₃, OCH(CH₃)₂, COOCH₃, COOCH₂CH₃, COOCH(CH₃)₂ or CN. Compounds of formula (I) more suitable for preparing a composition as a pharmaceutically active component are those compounds, in which Q represents a compound of formula (II) and R² represents 0 to 3 following identical or different substituents: F, Cl, Br, NO₂, OH, COOH, CF₃, CHF₂, CH₂F, N(CH₃)₂, N(CH₂CH₃)₂, N(CH₃)(CH₂CH₃), CH₃, CH₂CH₃, OCH₃, OCH₂CH₃, COOCH₃, COOCH₂CH₃ or CN. Compounds of formula (I) particularly suitable for preparing a composition as a pharmaceutically active component are those compounds, in which Q represents a compound of formula (II) and R² represents 0 to 3 following identical or different substituents: F, Cl, Br, NO₂, OH, COOH, CF₃, CHF₂, CH₂F, N(CH₃)₂, N(CH₂CH₃)₂, CH₃, CH₂CH₃, OCH₃, OCH₂CH₃ or CN.

Compounds of formula (I) suitable for preparing a composition as a pharmaceutically active component are those compounds, in which Q represents a compound of formula (III) and R¹ represents 0 to 4 identical or different substituents: F, Cl, OH, NO₂, CF₃, CH₃, CHF₂ or CH₂F. Compounds of formula (I) more suitable for preparing a composition as a pharmaceutically active component are those compounds, in which Q represents a compound of formula (III) and R¹ represents 0 to 2 identical or different substituents: F, Cl, OH, CH₃ or CF₃. Compounds of formula (I) particularly suitable for preparing a composition as a pharmaceutically active component are those compounds, in which Q represents a compound of formula (III) and R¹ represents 0 to 1 identical or different substituent: F or OH.

Compounds of formula (I) suitable for preparing a composition as a pharmaceutically active component are those compounds, in which X represents C₄₋₁₅ alkylene. Compounds of formula (I) more suitable for preparing a composition as a pharmaceutically active component are those compounds, in which X represents C₄₋₁₀ alkylene. Compounds of formula (I) particularly suitable for preparing a composition as a pharmaceutically active component are those compounds, in which X represents 1,4-butylidene, 1,5-pentylidene, 1,6-hexylidene, 1,7-heptylidene, 1,8-octylidene or 1,9-nonylidene.

Compounds of formula (I) suitable for preparing a composition as a pharmaceutically active component are those compounds, in which Y represents O, S, NH, -OC(O)-, -C(O)O-, -C(O)-, -NHC(O)- or -C(O)NH-. Compounds of formula (I) more suitable for preparing a composition as a pharmaceutically active component are those compounds, in which Y represents O.

In a composition comprising a compound of formula (I) in the invention, the molar ratio of the compound of formula (I) to the cyclodextrin, the cyclodextrin derivative or the surfactant with solubilization in the composition is the range of from 1:1000 to 200:1.

The representative compounds of a compound of formula (I) which can be prepared in a composition in the invention include, but are not limited to the following compounds:
1) N-cyano-N'-(6-phenoxyhexyl)-N"-(3-pyridyl)guanidine,
2) N-cyano-N'-(6-(p-chlorophenoxy)hexyl)-N"-(3-pyridyl)guanidine,
3) N-cyano-N'-(6-(o-chlorophenoxy)hexyl)-N"-(3-pyridyl)guanidine,
4) N-cyano-N'-(6-(o-methoxyphenoxy)hexyl)-N"-(3-pyridyl)guanidine,
5) N-cyano-N'-(6-(o-nitrophenoxy)hexyl)-N"-(3-pyridyl)guanidine,
6) N-cyano-N'-(6-(m-chlorophenoxy)hexyl)-N"-(3-pyridyl)guanidine,
7) N-cyano-N'-(6-(m-methoxyphenoxy)hexyl)-N"-(3-pyridyl)guanidine,
8) N-cyano-N'-(6-(m-nitrophenoxy)hexyl)-N"-(3-pyridyl)guanidine,
9) N-cyano-N'-(6-(p-fluorophenoxy)hexyl)-N"-(3-pyridyl)guanidine,
10) N-cyano-N'-(6-(m-fluorophenoxy)hexyl)-N"-(3-pyridyl)guanidine,
11) N-cyano-N'-(6-(o-fluorophenoxy)hexyl)-N"-(3-pyridyl)guanidine,
12) N-cyano-N'-(6-(p-fluorophenoxy)hexyl)-N"-(4-pyridyl)guanidine,
13) N-cyano-N'-(6-(m-fluorophenoxy)hexyl)-N"-(4-pyridyl)guanidine,
14) N-cyano-N'-(6-(o-fluorophenoxy)hexyl)-N"-(4-pyridyl)guanidine,
15) N-cyano-N'-(6-(p-methoxyphenoxy)hexyl)-N"-(3-pyridyl)guanidine,
16) N-cyano-N'-(6-(p-nitrophenoxy)hexyl)-N"-(3-pyridyl)guanidine,
17) N-cyano-N'-(6-(p-nitrophenoxy)hexyl)-N"-(4-pyridyl)guanidine,
18) N-cyano-N'-(6-(p-methoxyphenoxy)hexyl)-N"-(4-pyridyl)guanidine,
19) N-cyano-N'-(6-(p-chlorophenoxy)hexyl)-N"-(4-pyridyl)guanidine,
20) N-cyano-N'-(6-(m-nitrophenoxy)hexyl)-N"-(4-pyridyl)guanidine,
21) N-cyano-N'-(6-(m-methoxyphenoxy)hexyl)-N"-(4-pyridyl)guanidine,
22) N-cyano-N'-(6-(m-chlorophenoxy)hexyl)-N"-(4-pyridyl)guanidine,
23) N-cyano-N'-(6-(o-nitrophenoxy)hexyl)-N"-(4-pyridyl)guanidine,
24) N-cyano-N'-(6-(o-methoxyphenoxy)hexyl)-N"-(4-pyridyl)guanidine,
25) N-cyano-N'-(6-(o-chlorophenoxy)hexyl)-N"-(4-pyridyl)guanidine,
26) N-cyano-N'-(6-phenoxyhexyl)-N"-(4-pyridyl)guanidine,
27) N-cyano-N'-(6-(2,4-dichlorophenoxy)hexyl)-N"-(4-pyridyl)guanidine,
28) N-cyano-N'-(6-(2,4,5-trichlorophenoxy)hexyl)-N"-(4-pyridyl)guanidine,
29) N-cyano-N'-(7-phenoxyheptyl)-N"-(3-pyridyl)guanidine,
30) N-cyano-N'-(7-phenoxyheptyl)-N"-(4-pyridyl)guanidine,
31) N-cyano-N'-(7-(p-fluorophenoxy)heptyl)-N" -(3-pyridyl)guanidine,
32) N-cyano-N'-(7-(o-fluorophenoxy)heptyl)-N"-(3-pyridyl)guanidine,
33) N-cyano-N'-(7-(m-fluorophenoxy)heptyl)-N"-(3-pyridyl)guanidine,
34) N-cyano-N'-(7-(p-fluorophenoxy)heptyl)-N"-(4-pyridyl)guanidine,
35) N-cyano-N'-(7-(o-fluorophenoxy)heptyl)-N"-(4-pyridyl)guanidine,
36) N-cyano-N'-(7-(m-fluorophenoxy)heptyl)-N"-(4-pyridyl)guanidine
37) N-cyano-N'-(7-(o-chlorophenoxy)heptyl)-N"-(4-pyridyl)guanidine,
38) N-cyano-N'-(7-(o-chlorophenoxy)heptyl)-N"-(3-pyridyl)guanidine,
39) N-cyano-N'-(7-(o-methoxyphenoxy)heptyl)-N"-(3-pyridyl)guanidine,
40) N-cyano-N'-(7-(o-methoxyphenoxy)heptyl)-N"-(4-pyridyl)guanidine,
41) N-cyano-N'-(7-(o-nitrophenoxy)heptyl)-N"-(4-pyridyl)guanidine,
42) N-cyano-N'-(7-(o-nitrophenoxy)heptyl)-N"-(3-pyridyl)guanidine,
43) N-cyano-N'-(7-(m-chlorophenoxy)heptyl)-N"-(3-pyridyl)guanidine,
44) N-cyano-N'-(7-(m-chlorophenoxy)heptyl)-N"-(4-pyridyl)guanidine,
45) N-cyano-N'-(7-(m-methoxyphenoxy)heptyl)-N"-(4-pyridyl)guanidine,
46) N-cyano-N'-(7-(m-methoxyphenoxy)heptyl)-N"-(3-pyridyl)guanidine,
47) N-cyano-N'-(7-(m-nitrophenoxy)heptyl)-N"-(3-pyridyl)guanidine,
48) N-cyano-N'-(7-(m-nitrophenoxy)heptyl)-N"-(4-pyridyl)guanidine,
49) N-cyano-N'-(7-(p-chlorophenoxy)heptyl)-N"-(4-pyridyl)guanidine,
50) N-cyano-N'-(7-(p-chlorophenoxy)heptyl)-N"-(3-pyridyl)guanidine,
51) N-cyano-N'-(7-(p-methoxyphenoxy)heptyl)-N"-(3-pyridyl)guanidine,
52) N-cyano-N'-(7-(p-methoxyphenoxy)heptyl)-N"-(4-pyridyl)guanidine,
53) N-cyano-N'-(7-(p-nitrophenoxy)heptyl)-N"-(4-pyridyl)guanidine,
54) N-cyano-N'-(7-(p-nitrophenoxy)heptyl)-N"-(3-pyridyl)guanidine,
55) N-cyano-N'-(5-(p-fluorophenoxy)pentyl)-N"-(3-pyridyl)guanidine,
56) N-cyano-N'-(5-(o-fluorophenoxy)pentyl)-N"-(3-pyridyl)guanidine,
57) N-cyano-N'-(5-(m-fluorophenoxy)pentyl)-N"-(3-pyridyl)guanidine,
58) N-cyano-N'-(5-(p-fluorophenoxy)pentyl)-N"-(4-pyridyl)guanidine,
59) N-cyano-N'-(5-(o-fluorophenoxy)pentyl)-N"-(4-pyridyl)guanidine,
60) N-cyano-N'-(5-(m-fluorophenoxy)pentyl)-N"-(4-pyridyl)guanidine
61) N-cyano-N'-(5-(p-chlorophenoxy)pentyl)-N"-(3-pyridyl)guanidine,
62) N-cyano-N'-(5-phenoxypentyl)-N"-(3-pyridyl)guanidine,
63) N-cyano-N'-(5-phenoxypentyl)-N"-(4-pyridyl)guanidine,
64) N-cyano-N'-(5-(p-chlorophenoxy)pentyl)-N"-(4-pyridyl)guanidine,
65) N-cyano-N'-(5-(p-methoxyphenoxy)pentyl)-N"-(3-pyridyl)guanidine,
66) N-cyano-N'-(5-(o-chlorophenoxy)pentyl)-N"-(3-pyridyl)guanidine,
67) N-cyano-N'-(5-(m-chlorophenoxy)pentyl)-N"-(3-pyridyl)guanidine,
68) N-cyano-N'-(5-(p-methoxyphenoxy)pentyl)-N"-(4-pyridyl)guanidine,
69) N-cyano-N'-(5-(o-chlorophenoxy)pentyl)-N"-(4-pyridyl)guanidine,
70) N-cyano-N'-(5-(m-chlorophenoxy)pentyl)-N"-(4-pyridyl)guanidine
71) N-cyano-N'-(8-(p-fluorophenoxy)octyl)-N"-(3-pyridyl)guanidine,
72) N-cyano-N'-(8-(o-fluorophenoxy)octyl)-N"-(3-pyridyl)guanidine,
73) N-cyano-N'-(8-(m-fluorophenoxy)octyl)-N"-(3-pyridyl)guanidine,
74) N-cyano-N'-(8-(p-fluorophenoxy)octyl)-N"-(4-pyridyl)guanidine,
75) N-cyano-N'-(8-(o-fluorophenoxy)octyl)-N"-(4-pyridyl)guanidine,
76) N-cyano-N'-(8-(m-fluorophenoxy)octyl)-N"-(4-pyridyl)guanidine,
77) N-cyano-N'-(8-(p-chlorophenoxy)octyl)-N"-(4-pyridyl)guanidine,
78) N-cyano-N'-(8-(p-chlorophenoxy)octyl)-N"-(3-pyridyl)guanidine,
79) N-cyano-N'-(8-phenoxyoctyl)-N"-(3-pyridyl)guanidine,
80) N-cyano-N'-(8-phenoxyoctyl)-N"-(4-pyridyl)guanidine,
81) N-cyano-N'-(10-phenoxydecyl)-N"-(4-pyridyl)guanidine,
82) N-cyano-N'-(10-phenoxydecyl)-N"-(3-pyridyl)guanidine,
83) N-cyano-N'-(10-(p-chlorophenoxy)decyl)-N"-(3-pyridyl)guanidine,
84) N-cyano-N'-(10-(o-chlorophenoxy)decyl)-N"-(3-pyridyl)guanidine,
85) N-cyano-N'-(10-(m-chlorophenoxy)decyl)-N"-(3-pyridyl)guanidine,
86) N-cyano-N'-(10-(p-fluorophenoxy)decyl)-N"-(3-pyridyl)guanidine,
87) N-cyano-N'-(10-(m-fluorophenoxy)decyl)-N"-(3-pyridyl)guanidine,
88) N-cyano-N'-(10-(o-fluorophenoxy)decyl)-N"-(3-pyridyl)guanidine,
89) N-cyano-N'-(10-(p-methoxyphenoxy)decyl)-N"-(3-pyridyl)guanidine,
90) N-cyano-N'-(10-(m-methoxyphenoxy)decyl)-N"-(3-pyridyl)guanidine,
91) N-cyano-N'-(10-(o-methoxyphenoxy)decyl)-N"-(3-pyridyl)guanidine,
92) N-cyano-N'-(10-(p-nitrophenoxy)decyl)-N"-(3-pyridyl)guanidine,
93) N-cyano-N'-(10-(o-nitrophenoxy)decyl)-N"-(3-pyridyl)guanidine,
94) N-cyano-N'-(10-(m-nitrophenoxy)decyl)-N"-(3-pyridyl)guanidine,
95) N-cyano-N'-(10-(p-chlorophenoxy)decyl)-N"-(4-pyridyl)guanidine,
96) N-cyano-N'-(10-(o-chlorophenoxy)decyl)-N"-(4-pyridyl)guanidine,
97) N-cyano-N'-(10-(m-chlorophenoxy)decyl)-N"-(4-pyridyl)guanidine,
98) N-cyano-N'-(10-(p-fluorophenoxy)decyl)-N"-(4-pyridyl)guanidine,
99) N-cyano-N'-(10-(m-fluorophenoxy)decyl)-N"-(4-pyridyl)guanidine,
100) N-cyano-N'-(10-(o-fluorophenoxy)decyl)-N"-(4-pyridyl)guanidine,
101) N-cyano-N'-(10-(p-methoxyphenoxy)decyl)-N"-(4-pyridyl)guanidine,
102) N-cyano-N'-(10-(m-methoxyphenoxy)decyl)-N"-(4-pyridyl)guanidine,
103) N-cyano-N'-(10-(o-methoxyphenoxy)decyl)-N"-(4-pyridyl)guanidine,
104) N-cyano-N'-(10-(p-nitrophenoxy)decyl)-N"-(4-pyridyl)guanidine,
105) N-cyano-N'-(10-(o-nitrophenoxy)decyl)-N"-(4-pyridyl)guanidine,
106) N-cyano-N'-(10-(m-nitrophenoxy)decyl)-N"-(4-pyridyl)guanidine,
107) N-cyano-N'-(12-phenoxydodecyl)-N"-(4-pyridyl)guanidine,
108) N-cyano-N'-(12-phenoxydodecyl)-N"-(3-pyridyl)guanidine,
109) N-cyano-N'-(12-(p-chlorophenoxy)dodecyl)-N"-(3-pyridyl)guanidine,
110) N-cyano-N'-(12-(o-chlorophenoxy)dodecyl)-N"-(3-pyridyl)guanidine,
111) N-cyano-N'-(12-(m-chlorophenoxy)dodecyl)-N"-(3-pyridyl)guanidine,
112) N-cyano-N'-(12-(p-fluorophenoxy)dodecyl)-N"-(3-pyridyl)guanidine,
113) N-cyano-N'-(12-(m-fluorophenoxy)dodecyl)-N"-(3-pyridyl)guanidine,
114) N-cyano-N'-(12-(o-fluorophenoxy)dodecyl)-N"-(3-pyridyl)guanidine,
115) N-cyano-N'-(12-(p-methoxyphenoxy)dodecyl)-N"-(3-pyridyl)guanidine,
116) N-cyano-N'-(12-(m-methoxyphenoxy)dodecyl)-N"-(3-pyridyl)guanidine,
117) N-cyano-N'-(12-(o-methoxyphenoxy)dodecyl)-N"-(3-pyridyl)guanidine,
118) N-cyano-N'-(12-(p-nitrophenoxy)dodecyl)-N"-(3-pyridyl)guanidine,
119) N-cyano-N'-(12-(o-nitrophenoxy)dodecyl)-N"-(3-pyridyl)guanidine,
120) N-cyano-N'-(12-(m-nitrophenoxy)dodecyl)-N"-(3-pyridyl)guanidine,
121) N-cyano-N'-(12-(p-chlorophenoxy)dodecyl)-N"-(4-pyridyl)guanidine,
122) N-cyano-N'-(12-(o-chlorophenoxy)dodecyl)-N"-(4-pyridyl)guanidine,
123) N-cyano-N'-(12-(m-chlorophenoxy)dodecyl)-N"-(4-pyridyl)guanidine,
124) N-cyano-N'-(12-(p-fluorophenoxy)dodecyl)-N"-(4-pyridyl)guanidine,
125) N-cyano-N'-(12-(m-fluorophenoxy)dodecyl)-N"-(4-pyridyl)guanidine,
126) N-cyano-N'-(12-(o-fluorophenoxy)dodecyl)-N"-(4-pyridyl)guanidine,
127) N-cyano-N'-(12-(p-methoxyphenoxy)dodecyl)-N"-(4-pyridyl)guanidine,
128) N-cyano-N'-(12-(m-methoxyphenoxy)dodecyl)-N"-(4-pyridyl)guanidine,
129) N-cyano-N'-(12-(o-methoxyphenoxy)dodecyl)-N"-(4-pyridyl)guanidine,
130) N-cyano-N'-(12-(p-nitrophenoxy)dodecyl)-N"-(4-pyridyl)guanidine,
131) N-cyano-N'-(12-(o-nitrophenoxy)dodecyl)-N"-(4-pyridyl)guanidine,
132) N-cyano-N'-(12-(m-nitrophenoxy)dodecyl)-N"-(4-pyridyl)guanidine,
133) 1,12-bis(N'-cyano-N"-4-pyridylguanidine)dodecane,
134) N-(12-(tert-butoxycarbonylamino)dodecyl)-N'-cyano-N"-(4-pyridyl)guanidine,
135) N-(6-((4-chlorobenzoic acid)ester)hexyl)-N'-cyano-N"-(4-pyridyl)guanidine,
136) N-cyano-N'-(6-(2-chloro-4-fluorphenyl)oxo)hexyl)-N"-(4pyridyl)guanidine,
137) N-cyano-N'-(6-(o-bromophenoxy)hexyl)-N"-(4-pyridyl)guanidine,
138) N-cyano-N'-(6-((2,6-dichlorophenyl)oxo)hexyl)-N"-(4-pyridyl)guanidine,
139) N-cyano-N'-(6-((2,4,6-trichlorophenyl)oxo)hexyl)-N"-(4-pyridyl)guanidine,
140) N-cyano-N'-(6-(o-trifluoromethoxyphenyl)oxo)hexyl)-N"-(4-pyridyl)guanidine,
141) N-cyano-N'-(5-(o-chlorophenoxy)pentyl)-N"-(4-pyridyl)guanidine,
142) N-cyano-N'-(7-(o-chlorophenoxy)heptyl)-N"-(4-pyridyl)guanidine,
143) N-cyano-N'-(5-(p-chlorophenoxy)pentyl)-N"-(4-pyridyl)guanidine,
144) N-cyano-N'-(8-((o-methoxyphenyl)oxo)octyl)-N"-(4-pyridyl)guanidine,
145) N-cyano-N'-(5-((2,4-dichlorophenyl)oxo)pentyl)-N"-(4-pyridyl)guanidine,
146) N-cyano-N'-(5-((2,4,6-trichlorophenyl)oxo)pentyl)-N"-(4-pyridyl)guanidine,
147) (E)-N-(6-(4-chlorophenoxy)-hexen-3-yl)-N'-cyano-N"-(4-pyridyl)guanidine,
148) (Z)-N-cyano-N'-(6-phenylhexen-5-yl)-N"-(4-pyridyl)guanidine,
149) (E)-N-cyano-N'-(6-phenylhexen-5-yl)-N"-(4-pyridyl)guanidine,
150) (E,E)-N-cyano-N'-(6-phenylhexa-3,5-dienyl)-N"-(4-pyridyl)guanidine,
151) (Z)-N-cyano-N'-(6-(4-chlorophenyl)hexen-5-yl)-N"-(4-pyridyl)guanidine,
152) N-cyano-N'-(6-phenylhexyn-5-yl)-N"-(4-pyridyl)guanidine,
153) N-cyano-N'-(13-phenyltridecyn-12-yl)-N"-(4-pyridyl)guanidine,
154) N-cyano-N'-(6-phenylhexyn-5-yl)-N"-5-(2-methoxypyridyl)guanidine,
155) N-cyano-N'-(6-phenylhexyn-5-yl)-N"-3-(2-chloropyridyl)guanidine,
156) N-(6-benzoylaminohexyl)-N'-cyano-N"-4-pyridyl guanidine,
157) N-(6-(4-chlorobenzoylamino)hexyl)-N'-cyano-N"-4-pyridyl guanidine,
158) N-(6-(4-chlorobenzoylamino)hexyl)-N'-cyano-N"-(2-methoxy-5-pyridyl)guanidine,
159) N-(6-(4-chlorobenzoylamino)hexyl)-N'-cyano-N"-(2-methyl-4-pyridyl)guanidine,
160) N-cyano-N'-(5-phenylaminocarbonylpentyl)-N"-(4-pyridyl)guanidine,
161) N-(5-(4-chlorobenzoyl)pentyl)-N'-cyano-N"-(4-pyridyl)guanidine,
162) N-(7-(4-chlorobenzoyl)heptyl)-N'-cyano-N"-(4-pyridyl)guanidine,
163) N-(6-(4-chlorobenzoyl)hexyl)-N'-cyano-N"-(4-pyridyl)guanidine,
164) N-(6-(3-pyridyl-oxo)-1-hexyl)-N'-cyano-N"-(4-pyridyl)guanidine,
165) N-(6-(1-imidazolyl)-1-hexyl)-N'-cyano-N"-(4-pyridyl)guanidine,
166) N-(6-(2-quinolyl-oxo)-1-hexyl)-N'-cyano-N"-(4-pyridyl)guanidine,
167) N-(6-(3-pyridyl)-1-hexyl)-N'-cyano-N"-(4-pyridyl)guanidine,
168) N-(6-(3-quinolyl)-1-hexyl)-N'-cyano-N"-(4-pyridyl)guanidine,
169) N-(6-(5-pyrimidinyl)-1-hexyl)-N'-cyano-N"-(4-pyridyl)guanidine,
170) 1,12-bis(N'-cyano-N"-3-pyridyl guanidyl)-dodecane,
171) 1,10-bis(N'-cyano-N"-4-pyridylguanidine)decane,
172) 2,2'-bis(N'-cyano-N"-3-pyridyl guanidyl)diethyldisulfide, or
173) 1,4-bis(N'-cyano-N"-4-pyridylguanidinemethyl)cyclohexane.

The invention further includes compositions prepared with compounds in Chinese patent applications CN 98805480.9, CN 98805590.2, CN 98805591.0, CN 98805595.3, ZL 02810102.2 and CN 98805594.5 and a cyclodextrin and/or a cyclodextrin derivative.

Where a compound of formula (I) or a tautomer of a compound of formula (I) according to the invention has optical isomers, an R isomer, an S isomer, or a mixture of an R isomer and an S isomer can be used to prepare a composition.

Where a compound of formula (I) or a tautomer of compound of formula (I) according to the invention has diastereoisomers, a Z isomer, an E isomer, or a mixture of a Z isomer and an E isomer can be used to prepare a composition.

A pharmaceutically acceptable salt of a compound of formula (I) or a tautomer of a compound of formula (I) according to the invention refers to a salt prepared with a compound of formula (I) or a tautomer of a compound of formula (I) with a suitable acid, in which the acid includes, but is not limited to one or more than one of the following: hydrochloric acid, hydrobromic acid, hydroiodic acid, sulfuric acid, nitric acid, acetic acid, phosphoric acid, lactic acid, maleic acid, phthalic acid, citric acid, propionic acid, benzoic acid, glutaric acid, gluconic acid, methanesulfonic acid, salicylic acid, succinic acid, tartaric acid, methylbenzenesulfonic acid, aminosulfonic acid, fumaric acid, formic acid, malic acid, ascorbic acid, malonic acid, oxalic acid, mandelic acid, hydroxyacetic acid, benzenesulfonic acid, naphthalene sulfonic acid, trimesic acid and glutamic acid.

A pharmaceutically acceptable salt of a compound of formula (I) or a tautomer of a compound of formula (I) according to the invention can further form a solvate. The solvent with which the pharmaceutically acceptable salt of the compound of formula (I) or the tautomer of the compound of formula (I) can form a solvate is selected from one or more than one of the following: water, ethanol, glycerol, acetone, butanone and polyethylene glycol.

A solvate of a compound of formula (I) or a tautomer of a compound of formula (I) according to the invention refers to a solvate formed with a compound of formula (I) or a tautomer of a compound of formula (I) with one or more than one of the following: water, ethanol, glycerol, acetone, butanone and polyethylene glycol.

The composition comprising a compound of formula (I) disclosed in the invention can be prepared as a medicament for treating proliferative diseases, which include, but are not limited to various cancers, diseases or disorders induced by abnormal cell proliferation. More specifically, the proliferative diseases include, but are not limited to leukemia, acute granulocyte leukemia, chronic myelocytic leukemia, chronic myelocytic leukemia, myelodysplasia, multiple myeloma, Hodgkin's disease or non-Hodgkin's lymphoma, small cell or non-small cell lung cancer, gastric cancer, bowel cancer, colon cancer, rectal cancer, prostate cancer, ovarian cancer, breast cancer, brain cancer, head and neck cancer, urethral carcinoma, renal carcinoma, bladder cancer, melanoma, liver cancer, skin cancer, uterine cancer and pancreatic cancer.

The composition comprising a compound of formula (I) disclosed in the invention can also be used in combination with one or more than one compound to treat proliferative diseases. The composition of the invention can be used simultaneously or sequentially when combined with other compounds. Other compounds which can be used in combination with a composition comprising a compound of formula (I) provided in the invention to treating proliferative diseases include, but are not limited to triazine derivatives such as hexamethylmelamine; enzymes such as asparagine enzyme; antibacterial agents such as bleomycin, dactinomycin, daunorubicin, adriamycin, idarubicin, mitomucin, epirubicin or plicamycin; alkylating agents such as busulfan, carboplatin, carmustine, chlorambucil, cisplatin, cyclophosphamide, dacarbazine, isocyclophosphamide, lomustine, mustine, melphalan, procarbazine or thiotepa; metabolic antagonists such as cladribine, cytarabine, floxuridine, fludarabine, fluorouracil, hydroxyurea, mercaptopurine, methotrexate, gemcitabine, pentostatin or tioguanine; antimitotic agents such as etoposide, paclitaxel, teniposide, vinblastine, vinorelbine or vincristine; hormone drugs such as aromatic enzyme inhibitors e.g. aminoglutethimide; corticosteroids such as dexamethasone, prednison or luteinizing hormone releasing hormone (LH-RH); antiestrogenic agents such as tamaxin, formestane or letrozole; antiandrogen agents such as flutamide; biological response modifiers such as aldesleukin in lymphokine or other interleukins; interferons such as interferon-α; growth factors such as erythropoietin, neupogen or sargramostim; differentiation agents such as vitamin D derivatives e.g seocalcitol; all-trans retinoic acid; immunomodulators such as levamisol; monoclonal antibody; tumor necrosis factor α; or angiogenesis inhibitors. Other compounds more suitable to be used in combination with a composition comprising a compound of formula (I) disclosed in the invention to treat proliferative diseases are selected from one or more than one of the following: paclitaxel, fluorouracil, etoposide, cyclophosphamide, cisplatin, carboplatin, vincristine, gemcitabine, vinorelbine, chlorambucil, adriamycin, melphalan and seocalcitol.

Where a composition comprising a compound of formula (I) disclosed in the invention is used to treat proliferative diseases, the composition can also be used in combination with ionizing radiation.

Where a composition comprising a compound of formula (I) disclosed in the invention is used to treat proliferative diseases, the composition can also be used in combination with medicaments for alleviating side effects of antitumor therapy. The medicaments for alleviating side effects of antitumor therapy include, but are not limited to amifostine, leucovorin and mesna.

The invention further relates to a pharmaceutical preparation comprising a composition comprising a compound of formula (I). The preparation in the invention refers to an appropriate pharmaceutical formulation prepared with a composition comprising a compound of formula (I) alone or along with necessary pharmaceutically acceptable pharmaceutical excipients, such that the preparation is suitable to be applied via a route of administration including, but not limited to oral administration, buccal administration, intravenous administration, intraperitoneal injection, subcutaneous injection, intramuscular injection, nasal drops, eye drops, inhalation, anal administration, vaginal administration, epidermal administration and the like. The appropriate pharmaceutical formulation includes, but is not limited to infusion, water injection, powder injection, lyophilized powder injection, oral solution, syrup, tablet, pill, capsule, granules, gel, soft capsule, suppository, aerosol, cream and the like. The weight of the composition comprising a compound of formula (I) is about 0.1% to 100% by weight of the pharmaceutical preparation. The unit dosage contains about 0.1 mg to 5 g of a compound of formula (I), and more appropriate unit dosage contains about 0.1 mg to 3 g of a compound of formula (I). Unit dosage refers to a unit which can be applied to a patient and can be easily operated and packaged, *i.e.* a single dosage.

Where the composition comprising a compound of formula (I) disclosed in the invention is prepared as a lyophilized powder injection, a pharmaceutical freeze-dried excipient may be or may not be further added. The excipient is selected from one or more than one of the following: mannitol, sorbierite, sodium chloride, glucose, fructose, sucrose, xylitol and lactose.

Where the composition comprising a compound of formula (I) provided in the invention is prepared as a powder injection, a water injection or a lyophilized powder injection, an appropriate amount of a sterile pharmaceutical diluent, which include, but is not limited to water for injection, physiological saline, glucose water, and other known aqueous carriers, can be added prior to use to prepare a solution preparation for intramuscular injection or intravenous administration.

The invention also discloses a process for preparing a composition comprising a compound of formula (I).

Process 1 The process comprises dissolving a compound of formula (I) in a solvent, adding a cyclodextrin, a cyclodextrin derivative and/or a surfactant with solubilization, and then removing the solvent. The solvent used to dissolve the compound of formula (I) is selected from one or more than one of the following: chloroform, dichloromethane, glycerol, methanol, ethanol, propanol, isopropanol, acetone, tetrahydrofuran, dimethylformamide, dimethylsulfoxide, butanol, ethyl acetate, ethyl formate, formic acid, acetic acid, trifluoroacetic acid, propylene glycol, polyethylene glycol 200, polyethylene glycol 400, polyethylene glycol 600 and an acidic solution, wherein the acidic solution is selected from the group consisting of hydrochloric acid solution, sulfuric acid solution, nitric acid solution, phosphoric acid solution, formic acid solution, acetic acid solution, methanesulfonic acid solution, benzenesulfonic acid solution, methylbenzenesulfonic acid solution, nitro benzenesulfonic acid solution, ethyl sulfonic acid solution, propanesulfonic acid solution, tris solution, naphthalene sulfonic acid solution and citric acid solution.

Process 2 The process comprises dissolving a compound of formula (I) in a solvent, mixing with a solution of a cyclodextrin, a cyclodextrin derivative and/or a surfactant with solubilization, and then removing the solvent. The solvent used to dissolve the compound of formula (I) is selected from one or more than one of the following: chloroform, dichloromethane, glycerol, methanol, ethanol, propanol, isopropanol, acetone, tetrahydrofuran, dimethylformamide, dimethylsulfoxide, butanol, ethyl acetate, ethyl formate, formic acid, acetic acid, trifluoroacetic acid, propylene glycol, polyethylene glycol 200, polyethylene glycol 400, polyethylene glycol 600, water and an acidic solution, wherein the acidic solution is selected from a group consisting of hydrochloric acid solution, sulfuric acid solution, nitric acid solution, phosphoric acid solution, formic acid solution, acetic acid solution, methanesulfonic acid solution, benzenesulfonic acid solution, toluenesulfonic acid solution, nitro benzenesulfonic acid solution, ethyl sulfonic acid solution, propanesulfonic acid solution, tris solution, naphthalene sulfonic acid solution and citric acid solution, The solvent used to dissolve the cyclodextrin, cyclodextrin derivatives and/or surfactant with solubilization is selected from one or more than one of the following: water, glycerol, methanol, ethanol, propanol, isopropanol, butanol, chloroform, dichloromethane, acetone, tetrahydrofuran, dimethylformamide, dimethylsulfoxide, ethyl acetate, ethyl formate, formic acid, acetic acid, trifluoroacetic acid, propylene glycol, polyethylene glycol 200, polyethylene glycol 400, polyethylene glycol 600 and an acidic solution, wherein the acidic solution is selected from one or more than one of the following: hydrochloric acid solution, sulfuric acid solution, nitric acid solution, phosphoric acid solution, formic acid solution, acetic acid solution, methanesulfonic acid solution, benzenesulfonic acid solution, toluenesulfonic acid solution, nitro benzenesulfonic acid solution, ethyl sulfonic acid solution, propanesulfonic acid solution, tris solution, naphthalene sulfonic acid solution or citric acid solution.

Process 3 The process comprises adding a compound of formula (I) into a solution comprising a cyclodextrin, a cyclodextrin derivatives and/or a surfactant with solubilization, and then removing the solvent. The solvent used to dissolve the cyclodextrin, cyclodextrin derivatives and/or surfactant with solubilization is selected from one or more than one of the following: water, glycerol, methanol, ethanol, propanol, isopropanol, butanol, chloroform, dichloromethane, acetone, tetrahydrofuran, dimethylformamide, dimethylsulfoxide, butanol, ethyl acetate, ethyl formate, formic acid, acetic acid, trifluoroacetic acid, propylene glycol, polyethylene glycol 200, polyethylene glycol 400, polyethylene glycol 600 and an acidic solution, wherein the acidic solution is selected from a group consisting of one or more mixed liquor of hydrochloric acid solution, sulfuric acid solution, nitric acid solution, phosphoric acid solution, formic acid solution, acetic acid solution, methanesulfonic acid solution, benzenesulfonic acid solution, toluenesulfonic acid solution, nitro benzenesulfonic acid solution, ethyl sulfonic acid solution, propanesulfonic acid solution, tris solution, naphthalene sulfonic acid solution and citric acid solution.

The feeding molar ratio of a compound of formula (I) to a cyclodextrin or a cyclodextrin derivative is in the range of from 1:0.1 to 1:1000. More appropriate feeding molar ratio of a compound of formula (I) to a cyclodextrin or a cyclodextrin derivative is in the range of from 1:1 to 1:100.

The feeding molar ratio of a compound of formula (I) to a surfactant with solubilization is in the range of from 1:0.001 to 1:1000. More appropriate feeding molar ratio of a compound of formula (I) to a surfactant with solubilization is in the range of from 1:0.005 to 1:100.

In specific embodiments, such as Example 2 and Example 3, HP-β-CD is added during the preparation of a composition and a clear solution can be obtained when the lyophilized powder of the resultant composition is redissolved in Example 2. However, when the conditions in Example 3 are the same as those in Example 2 except that HP-β-CD is not added during the preparation of a composition, the resultant lyophilized powder cannot be redissolved. Therefore, the addition of HP-β-CD can significantly increase the solubility of the compound in Example 2. Similarly, in Example 13 and Example 14, a clear solution can be obtained when the resultant composition is redissolved in Example 14 after adding HP-β-CD during the preparing process in Example 13, while the lyophilized powder in Example 13 cannot be redissolved. Different kinds of cyclodextrins or cyclodextrin derivatives can solubilize the same compound and improve the solubilities of compositions prepared with various processes. Therefore, the composition comprising a compound of formula (I) provided in the invention can significantly improve the solubility of a compound of formula (I) in an aqueous solution.

In order to better illustrate the technical solutions of the invention, the most commonly used HP-β-CD, SBE-β-CD, polysorbate 80, polyethylene glycol-12-hydroxystearate and polyoxyethylene castor oil are used in the following examples. However, the invention is not limited thereby.

### ABBREVIATIONS:

- CD:: cyclodextrin
- HP-β-CD:: hydroxypropyl-β-cyclodextrin
- SDS:: sodium dodecylsulphate
- mM:: mmol/l
- SBE7-β-CD:: sulfobutylether-β-cyclodextrin with a mean substitution of 7
- SBE6.5-β-CD:: sulfobutylether-β-cyclodextrin with a mean substitution of 6.5
- SBE6-β-CD:: sulfobutylether-β-cyclodextrin with a mean substitution of 6
- SBE-β-CD:: sulfobutylether-β-cyclodextrin
- 0.1N:: 0.1 mol/l

### SPECIFIC EXAMPLES

Solubilization tests are carried out with the following compounds in the invention:
Compound 1:
   N-cyano-N'-(6-(p-chlorophenoxy)hexyl)-N"-(4-pyridyl)guanidine,
Compound 2:
   N-cyano-N'-(6-(o-chlorophenoxy)hexyl)-N" -(4-pyridyl)guanidine,
Compound 3:
   N-cyano-N'-(6-(o-methoxyphenoxy)hexyl)-N"-(4-pyridyl)guanidine,
Compound 4: 1,12-bis(N'-cyano-N"-4-pyridylguanidine)dodecane,
Compound 5:
   N-(12-(tert-butoxycarbonylamino)dodecyl)-N'-cyano-N"-(4-pyridyl)guanidine,
Compound 6:
   N-(6-((4-chlorobenzoic acid)ester)hexyl)-N'-cyano-N"-(4-pyridyl)guanidine,
Compound 7:
   N-cyano-N'-(6-(2-chloro-4-fluorophenyl)oxo)hexyl)-N"-(4-pyridyl)guanidine,
Compound 8:
   N-cyano-N'-(6-(o-bromophenoxy)hexyl)-N"-(4-pyridyl)guanidine,
Compound 9:
   N-cyano-N'-(6-((2,6-dichlorophenyl)oxo)hexyl)-N"-(4-pyridyl)guanidine,
Compound 10:
   ) N-cyano-N'-(6-(o-trifluoromethoxyphenyl)oxo)hexyl)-N"-(4-pyridyl)guanidine,
Compound 11:
   N-cyano-N'-(5-(o-chlorophenoxy)pentyl)-N"-(4-pyridyl)guanidine,
Compound 12:
   N-cyano-N'-(7-(o-chlorophenoxy)heptyl)-N"-(4-pyridyl)guanidine,
Compound 13:
   N-cyano-N'-(5-(p-chlorophenoxy)pentyl)-N"-(4-pyridyl)guanidine,
Compound 14:
   N-cyano-N'-(8-((o-methoxyphenyl)oxo)octyl)-N"-(4-pyridyl)guanidine,
Compound 15:
   N-cyano-N'-(5-((2,4-dichlorophenyl)oxo)pentyl)-N"-(4-pyridyl)guanidine,
Compound 16:
   N-cyano-N'-(5-((2,4,6-trichlorophenyl)oxo)pentyl)-N"-(4-pyridyl)guanidine,
Compound 17:
   N-cyano-N'-(6-((2,4,6-trichlorophenyl)oxo)hexyl)-N"-(4-pyridyl)guanidine

### Example 1 composition comprising compound 1 and HP-β-CD (molar ratio of 1:2)

2.5 mg of compound 1 was weighed and dissolved with 0.5 ml of 0.1N hydrochloric acid solution. 21 mg of HP-β-CD was dissolved in the resultant solution. A clear solution was obtained. The solvent was removed by freeze-drying to obtain the desired composition 1.

### Example 2 composition comprising compound 1 and HP-β-CD (molar ratio of 1:2)

2.5 mg of compound 1 was weighed and dissolved with 0.5 ml of 0.1N hydrochloric acid solution. 21 mg of HP-β-CD was dissolved in the resultant solution. A clear solution was obtained. The pH of the solution was adjusted with 0.1N of sodium hydroxide to such an extent that precipitates were just to generate. The solvent was removed by freeze-drying to obtain the desired composition 2.

### Example 3

2.5 mg of compound 1 was weighed and dissolved with 0.5 ml of 0.1N hydrochloric acid solution. A clear solution was obtained. An amount of sodium hydroxide solution which was equal to that in Example 2 was added. The solvent was removed by freeze-drying to obtain the desired composition 3.

### Compositions in Examples 4 to 24 were prepared with the preparation process of Example 2.

2 mg of compound 1 was weighed and dissolved with 2 ml methanol and 0.5 ml DMSO to obtain Control Solution 1.

2 mg of compound 2 was weighed and dissolved with 2 ml methanol and 0.5 ml DMSO to obtain Control Solution 2.

2 mg of compound 3 was weighed and dissolved with 2 ml methanol and 0.5 ml DMSO to obtain Control Solution 3.

After the compositions obtained in Examples 2 and 4-10 were dissolved in water, the concentrations of the composition solutions obtained in each example were determined by HPLC with Control Solution 1 as control.

After the compositions obtained in Examples 12-17 were dissolved in water, the concentrations of the composition solutions obtained in each example were determined by HPLC with Control Solution 2 as control.

After the compositions obtained in Examples 18-24 were dissolved in water, the concentrations of the composition solutions obtained in each example were determined by HPLC with Control Solution 3 as control.

Unless otherwise specified, the HPLC conditions used in the invention are as follows:
Agilent 1200;
Mobile phase: water (12.5 mM K₂BPO₄, 10 mM SDS, 13% acetonitrile with pH of 2.5 adjusted with phosphoric acid) : acetonitrile = 60:40, and the gradient changes from the initial ratio of 60:40 to 34:66 in 10 min;
Chromatographic column: PLATISIL^{™} ODS 5µ 150×4.6mm;
Wavelength of detection: 278 nm;
Flow rate: 1ml/min;
Column temperature: 40 °C.

Table 1 lists the concentrations of the solutions after the compositions obtained in each exampls were redissolved.

**Table 1. Redissolution of compositions in Examples 2 to 24**

| Numbering | Compounds of formula (I) | Types of cyclodextrins | Compounds of formula (I) : Cyclodextrins (molar ratio) | Concentrations after redissolution (mg/ml) phenomena | pH after redissolution |
|---|---|---|---|---|---|
| example 2 | compound 1 | HP-β-CD | 1:2 | 0.69 clear solution | 3.15 |
| example 3 | compound 1 | No cyclodextrin was added | | insoluble | 2.76 |
| example 4 | compound 1 | HP-β-CD | 1:3 | 0.71 clear solution | 3.62 |
| example 5 | compound 1 | HP-β-CD | 1:4 | 0.71 clear solution | 3.69 |
| example 6 | compound 1 | HP-β-CD | 1:6 | 0.71 clear solution | 4.06 |
| example 7 | compound 1 | SBE7-β-CD | 1:2 | 0.70 clear solution | 3.76 |
| example 8 | compound 1 | SBE7-β-CD | 1:3 | 0.71 clear solution | 3.74 |
| example 9 | compound 1 | SBE7-β-CD | 1:4 | 0.70 clear solution | 3.70 |
| example 10 | compound 1 | SBE7-β-CD | 1:6 | 0.73 clear solution | 3.50 |
| example 11 | compound 2 | No cyclodextrin was added | | insoluble | 3.45 |
| example 12 | compound 2 | HP-β-CD | 1:2 | 0.71 clear solution | 3.43 |
| example 13 | compound 2 | HP-β-CD | 1:4 | 0.95 clear solution | 3.91 |
| example 14 | compound 2 | HP-β-CD | 1:6 | 0.98 clear solution | 3.85 |
| example 15 | compound 2 | SBE7-β-CD | 1:2 | 0.97 clear solution | 3.52 |
| example 16 | compound 2 | SBE7-β-CD | 1:4 | 0.97 clear solution | 3.57 |
| example 17 | compound 2 | SBE7-β-CD | 1:6 | 0.98 clear solution | 3.78 |
| example 18 | compound 3 | HP-β-CD | 1:2 | 0.74 clear solution | 4.52 |
| example 19 | compound 3 | HP-β-CD | 1:4 | 0.94 clear solution | 4.88 |
| example 20 | compound 3 | HP-β-CD | 1:6 | 0.99 clear solution | 5.05 |
| example 21 | compound 3 | HP-β-CD | 1:8 | 0.94 clear solution | 5.14 |
| example 22 | compound 3 | SBE7-β-CD | 1:2 | 0.91 clear solution | 5.48 |
| example 23 | compound 3 | SBE7-β-CD | 1:4 | 1.09 clear solution | 5.90 |
| example 24 | compound 3 | SBE7-β-CD | 1:6 | 1.07 clear solution | 5.99 |

### Example 25 solubilization of compound 1 with polysorbate 80

2.5 mg of compound 1 was weighed and dissolved with 0.5 ml of 0.1N hydrochloric acid solution. 5% (V/V) of polysorbate 80 was added in the resultant solution. The pH of the solution was adjusted with 0.1N of sodium hydroxide to such an extent that precipitates were just to generate. The solution was placed for observation and it was found that the solution kept clear for 12 hours.

### Example 26 solubilization control of compound 1 with polysorbate 80

2.5 mg of compound 1 was weighed and dissolved with 0.5 ml of 0.1N hydrochloric acid solution. An amount of 0.1 N sodium hydroxide solution which is equal to that in Example 25 was added. Precipitates produced. The solution was placed for observation and it was found that the precipitates increased during 12 hours.

### Example 27 solubilization of compound 1 with polyethylene glycol-12-hydroxy stearate

2 mg of compound 1 was weighed and dissolved with 0.5 ml of 0.1N hydrochloric acid solution. 5% (V/V) of polyethylene glycol-12-hydroxy stearate was added in the resultant solution. The pH of the solution was adjusted with 0.1N of sodium hydroxide to such an extent that precipitates were just to generate. The solution was placed for observation and it was found that the solution kept clear for 12 hours.

### Example 28 solubilization control of compound 1 with polyethylene glycol-12-hydroxy stearate

2 mg of compound 1 was weighed and dissolved with 0.5 ml of 0.1N hydrochloric acid solution. The pH was adjusted with an amount of 0.1 N sodium hydroxide solution which is equal to that in Example 27. Precipitates produced. The solution was placed for observation and it was found that the precipitates increased during 12 hours.

### Example 29 solubilization of compound 1 with polyoxyethylene castor oil

2 mg of compound 1 was weighed and dissolved with 0.5 ml of 0.1N hydrochloric acid solution. 5% (V/V) of polyoxyethylene castor oil was added in the resultant solution. The pH of the solution was adjusted with 0.1N of sodium hydroxide to such an extent that precipitates were just to generate. The solution was placed for observation and it was found that the solution kept clear for 12 hours.

### Example 30 solubilization control of compound 1 with polyoxyethylene castor oil

2 mg of compound 1 was weighed and dissolved with 0.5 ml of 0.1N hydrochloric acid solution. The pH was adjusted with an amount of 0.1 N sodium hydroxide solution which is equal to that in Example 29. Precipitates produced. The solution was placed for observation and it was found that precipitates increased during 12 hours.

### Example 31 composition comprising compound 4 and HP-β-CD (molar ratio of 1:3)

2 mg/ml of a clear solustion was prepared with 19.4 mg of compound 4 and 0.1N of hydrochloric acid. 3 folds of molar weight of HP-β-CD was added in the resultant solution. The pH was adjusted to about 3 to 4 with 0.1N NaOH solution. The solvent was removed by freeze-drying to obtain the desired composition 31.

### Compositions in Examples 32 to 51 were prepared with the preparation process of Example 31.

Example 32 composition comprising compound 4 and SBE7-β-CD (molar ratio of 1:3)
Example 33 composition comprising compound 5 and HP-β-CD (molar ratio of 1:3)
Example 34 composition comprising compound 5 and SBE7-β-CD (molar ratio of 1:3)
Example 35 composition comprising compound 6 and HP-β-CD (molar ratio of 1:3)
Example 36 composition comprising compound 6 and SBE7-β-CD (molar ratio of 1:3)
Example 37 composition comprising compound 7 and HP-β-CD (molar ratio of 1:3)
Example 38 composition comprising compound 8 and HP-β-CD (molar ratio of 1:3)
Example 39 composition comprising compound 8 and SBE7-β-CD (molar ratio of 1:3)
Example 40 composition comprising compound 9 and HP-β-CD (molar ratio of 1:3)
Example 41 composition comprising compound 9 and SBE7-β-CD (molar ratio of 1:3)
Example 42 composition comprising compound 10 and HP-β-CD (molar ratio of 1:3)
Example 43 composition comprising compound 10 and SBE7-β-CD (molar ratio of 1:3)
Example 44 composition comprising compound 11 and HP-β-CD (molar ratio of 1:3)
Example 45 composition comprising compound 11 and SBE7-β-CD (molar ratio of 1:3)
Example 46 composition comprising compound 12 and HP-β-CD (molar ratio of 1:3)
Example 47 composition comprising compound 12 and SBE7-β-CD (molar ratio of 1:3)
Example 48 composition comprising compound 13 and HP-β-CD (molar ratio of 1:3)
Example 49 composition comprising compound 13 and SBE7-β-CD (molar ratio of 1:3)
Example 50 composition comprising compound 14 and HP-β-CD (molar ratio of 1:3)
Example 51 composition comprising compound 14 and SBE7-β-CD (molar ratio of 1:3)

### Example 52 determination of solubility of compound 4 in hydrochloric acid solution

1.6 mg of compound 4 was weighed and dissolved in a mixed solution of 1ml of methanol and 0.5 ml of DMSO to obtain a control solution. 7.66 mg of compound 4 was dissolved in hydrochloric acid solution of pH 4.0 and shaken in an ultrasonic device for 1 hour to prepare a saturated solution. The solubility of compound 4 in hydrochloric acid solution of pH 4.0 was determined by HPLC.

The solubilities of compound 5 to compound 17 in hydrochloric acid solution were determined with the process for determining the solubility in Example 52. The results were listed in Table 2.

After the freeze-dried compositions in Example 31 to Example 51 were redissolved in water, the control solutions used in determination of the solubilities of compound 14 to compound 17 were used as control respectively. The concentrations of the resultant solutions were determined by HPLC. The results were listed in Table 3.

**Table 2. Solubility of each compound in hydrochloric acid solution of pH 4.0 (Unit: mg/ml)**

| Compounds | Solubilities in hydrochloric acid of pH 4 | Compounds | Solubilities in hydrochloric acid of pH 4 |
|---|---|---|---|
| compound 4 | 0.00052 | compound 11 | 0.00285 |
| compound 5 | 0.00092 | compound 12 | 0.00372 |
| compound 6 | 0.00225 | compound 13 | 0.02952 |
| compound 7 | 0.09993 | compound 14 | 0.14812 |
| compound 8 | 0.20548 | compound 15 | 0.000829 |
| compound 9 | 0.05331 | compound 16 | 0.01753 |
| compound 10 | 0.01965 | compound 17 | 0.00669 |

**Table 3. Concentration of redissolved solution of each composition in Example 31 to Example 51 (Unit: mg/ml)**

| Examples | Concentrations | Folds of solubilization relative to each corresponding compound Folds |
|---|---|---|
| Example 31 | 1.677 | 3220 |
| Example 32 | 1.647 | 3162 |
| Example 33 | 1.685 | 1830 |
| Example 34 | 1.726 | 1874 |
| Example 35 | 3.081 | 1369 |
| Example 36 | 2.167 | 963 |
| Example 37 | 1.96 | 19.6 |
| Example 38 | 1.587 | 7.7 |
| Example 39 | 1.632 | 7.9 |
| Example 40 | 1.362 | 26 |
| Example 41 | 1.5 | 28 |
| Example 42 | 1.607 | 82 |
| Example 43 | 1.702 | 87 |
| Example 44 | 0.014 | 5.1 |
| Example 45 | 1.53 | 537 |
| Example 46 | 2.295 | 617 |
| Example 47 | 2.323 | 625 |
| Example 48 | 1.279 | 43 |
| Example 49 | 0.132 | 4.5 |
| Example 50 | 0.482 | 3.3 |
| Example 51 | 0.703 | 4.7 |

### Example 53 composition comprising compound 6 and SBE7-β-CD (1:2)

Compound 6 was dissolved with methanol and SBE7-β-CD was dissolved with water. The two resultant solutions were mixed in a ratio of compound 6:SBE7-β-CD =1:2 (molar ratio). The solvent was rotary evaporated to obtain the desired composition 53.

### Example 54 composition comprising compound 6 and SBE7-β-CD (1:4)

Compound 6 was dissolved with methanol and SBE7-β-CD was dissolved with water. The two resultant solutions were mixed in a ratio of compound 6:SBE7-β-CD =1:4 (molar ratio). The solvent was rotary evaporated to obtain the desired composition 54.

### Example 55 composition comprising compound 6 and HP-β-CD (1:4)

Compound 6 was dissolved with methanol and HP-β-CD was dissolved with water.The two resultant solutions were mixed in a ratio of compound 6: HP-β-CD =1:4 (molar ratio). The solvent was rotary evaporated to obtain the desired composition 55.

### Example 56 composition comprising compound 6 and HP-β-CD (1:4)

Compound 6 was dissolved with methanol and HP-β-CD was dissolved with ethanol. The two resultant solutions were mixed in a ratio of compound 6: HP-β-CD =1:4 (molar ratio). The solvent was rotary evaporated to obtain the desired composition 56.

### Example 57 composition comprising compound 6 and SBE7-β-CD (1:4)

Compound 6 was dissolved with methanol and SBE7-β-CD was dissolved with water. The two resultant solutions were mixed in a ratio of compound 6: SBE7-β-CD = 1:4 (molar ratio). After the mixture was placed at room temperature for 48 hours, the solvent was rotary evaporated to obtain the desired composition 57.

### Example 58 composition comprising compound 6 and HP-β-CD (1:4)

Compound 6 was dissolved with methanol and SBE7-β-CD was dissolved with water. The two resultant solutions were mixed in a ratio of compound 6: SBE7-β-CD = 1:4 (molar ratio). After the mixture was placed at room temperature for 48 hours, the solvent was rotary evaporated to obtain the desired composition 58.

### Example 59 composition comprising compound 6 and HP-β-CD (1:4)

Compound 6 was dissolved with methanol and HP-β-CD was dissolved with ethanol. The two resultant solutions were mixed in a ratio of compound 6: HP-β-CD =1:4 (molar ratio). After the mixture was placed at room temperature for 48 hours, the solvent was rotary evaporated to obtain the desired composition 59.

### Example 60 composition comprising compound 6 and HP-β-CD (1:4)

HP-β-CD was dissolved woth ethanol. Compound 6 was added into the resultant solution in a ratio of compound 6: HP-β-CD =1:4 (molar ratio). The solvent was rotary evaporated to obtain the desired composition 60.

After the compositions in Example 53 to Example 60 were redissolved in water, the control solution used in determination of the solubility of compound 6 was used as control. The concentrations of the resultant solutions were determined by HPLC. The results were listed in Table 4.

**Table 4. Concentration of the redissolved solution of each composition in Example 53 to Example 60 (Unit: µg/ml)**

| Examples | Concentrations | Folds of solubilization relative to compound 6 |
|---|---|---|
| Example 53 | 3.1 | 1.38 |
| Example 54 | 9.9 | 4.4 |
| Example 55 | 4.8 | 2.13 |
| Example 56 | 10.9 | 4.84 |
| Example 57 | 20.6 | 9.16 |
| Example 58 | 42.4 | 18.84 |
| Example 59 | 10.5 | 4.67 |
| Example 60 | 41 | 18.2 |

### Example 61 composition comprising compound 15 and HP-β-CD (1:3)

5.41 mg of compound 15 was dissolved with 2.705 ml 0.1N hydrochloric acid. To 1 ml of the resultant solution was added 3 folds of molar weight of HP-β-CD (22.86 mg). The pH of the solution was adjusted to about 3 to 4 with sodium hydroxide. The solvent was removed by freeze-drying to obtain the desired composition 61.

### Example 62 composition comprising compound and 15 SBE7-β-CD (1:3)

5.41 mg of compound 15 was dissolved with 2.705 ml 0.1N hydrochloric acid. To 1 ml of the resultant solution was added 3 folds of molar weight of SBE7-β-CD (33.08mg). The pH of the solution was adjusted to about 3 to 4 with sodium hydroxide. The solvent was removed by freeze-drying to obtain the desired composition 62.

### Example 63 composition comprising compound 15 and HP-β-CD

4.8 mg of compound 15 was dissolved with 2 ml of acetone to obtain solution A. 246 mg of HP-β-CD was dissolved with 1ml of water to obtain solution B. 55.76 µl of solution B was mixed with 0.5 ml of solution A. The solvent was rotary evaporated to obtain the desired composition 63 comprising compound 15:HP-β-CD=1:3 (molar ratio).

### Example 64 composition comprising compound 15 and HP-β-CD

2.49 mg of compound 15 was dissolved with 2 ml of ethanol to obtain solution A. 246 mg of HP-β-CD was dissolved with 1ml of water to obtain solution B. 28.92 µl of solution B was mixed with 0.5 ml of solution A. The solvent was rotary evaporated to obtain the desired composition 64 comprising compound 15:HP-β-CD=1:3 (molar ratio).

### Example 65 composition comprising compound 15 and HP-β-CD

4.09 mg of compound 15 was dissolved with 2 ml of methanol to obtain solution A. 246 mg of HP-β-CD was dissolved with 1ml of water to obtain solution B. 47.51 µl of solution B was mixed with 0.5 ml of solution A. The solvent was rotary evaporated to obtain the desired composition 65 comprising compound 15:HP-β-CD=1:3 (molar ratio).

### Example 66 composition comprising compound 15 and SBE7-β-CD

4.8 mg of compound 15 was dissolved with acetone to obtain solution A. 300 mg of SBE7-β-CD was dissolved with 1ml of water to obtain solution B. 66.15 µl of solution B was mixed with 0.5 ml of solution A. The solvent was rotary evaporated to obtain the desired composition 66 comprising compound 15:SBE7-β-CD=1:3 (molar ratio).

### Example 67 composition comprising compound 15 and SBE7-β-CD

2.49 mg of compound 15 was dissolved with ethanol to obtain solution A. 300 mg of SBE7-β-CD was dissolved with 1ml of water to obtain solution B. 34.32 µl of solution B was mixed with 0.5 ml of solution A. The solvent ws rotary evaporated to obtain the desired composition 67 comprising compound 15:SBE7-β-CD=1:3 (molar ratio).

### Example 68 composition comprising compound 15 and SBE7-β-CD

4.09 mg of compound 15 was dissolved with 2 ml of methanol. 300 mg of SBE7-β-CD was dissolved with 1ml of water to obtain solution B. 56.37 µl of solution B was mixed with 0.5 ml of solution A. The solvent was rotary evaporated to obtain the desired composition 68 comprising compound 15:SBE7-β-CD=1:3 (molar ratio).

### Example 69 composition comprising compound and 15 HP-β-CD

4.8 mg of compound 15 was dissolved with 2 ml acetone to obtain solution A. 245 mg of HP-β-CD was dissolved with 1ml of ethanol to obtain solution B. 56.0 µl of solution B was mixed with 0.5 ml of solution A. The solvent was rotary evaporated to obtain the desired composition 69 comprising compound 15:HP-β-CD=1:3 (molar ratio).

### Example 70 composition comprising compound and 15 HP-β-CD

2.49 mg of compound 15 was dissolved with 2 ml ethanol to obtain solution A. 245 mg of HP-β-CD was dissolved with 1ml of ethanol to obtain solution B. 29.04 µl of solution B was mixed with 0.5 ml of solution A. The solvent was rotary evaporated to obtain the desired composition 70 comprising compound 15:HP-β-CD=1:3 (molar ratio).

### Example 71 composition comprising compound and 15 HP-β-CD

4.09 mg of compound 15 was dissolved with 2 ml methanol to obtain solution A. 245 mg of HP-β-CD was dissolved with 1ml of ethanol to obtain solution B. 47.7 µl of solution B was mixed with 0.5 ml of solution A. The solvent was rotary evaporated to obtain the desired composition 71 comprising compound 15:HP-β-CD=1:3 (molar ratio).

After the compositions in Example 61 to Example 71 were redissolved in water, the control solution used in determination of the solubility of compound 15 was used as control. The concentrations of the obtained solutions were determined by HPLC. The results were listed in Table 5.

**Table 5. Concentration of redissolved solution of each composition in Example 61 to Example 71 (Unit: µg/ml)**

| Examples | Concentrations | Folds of solubilization relative to compound 15 |
|---|---|---|
| Example 61 | 812.333 | 980 |
| Example 62 | 897.055 | 1082 |
| Example 63 | 9.697 | 12 |
| Example 64 | 73.052 | 88 |
| Example 65 | 13.36 | 16 |
| Example 66 | 125.795 | 152 |
| Example 67 | 54.239 | 65 |
| Example 68 | 88.044 | 106 |
| Example 69 | 11.599 | 14 |
| Example 70 | 3.827 | 5 |
| Example 71 | 6.89 | 8 |

### Example 72 composition comprising compound 15 and SBE6.5-β-CD

4.86 mg of compound 15 was weighed and dissolved with 0.1M of hydrochloric acid to prepare 2 mg/ml of solution. 31.73 mg (about 3 folds of molar weight) of SBE6.5-β-CD was added into the resultant solution with vortexing. 0.1M of sodium hydroxide solution was dropwise added to such an extent that precipitates were just to generate and at the same time the precipitates can disappear. The solvent was removed by freeze-drying to obtain the desired composition 72. To 2 ml of water was added the resultant composition to obtain a clear solution.

### Example 73 composition comprising compound 4 and SBE6.5-β-CD

3.57 mg of compound 4 was weighed and dissolved with 0.1M of hydrochloric acid to prepare 2 mg/ml of solution. 27.68 mg (about 3 folds of molar weight) of SBE6.5-β-CD was added into the resultant solution with vortexing. 0.1M of sodium hydroxide solution was dropwise added to such an extent that precipitates were just to generate and at the same time the precipitates can disappear. The solvent was removed by freeze-drying to obtain the desired composition 73. To 2 ml of water was added the resultant composition into to obtain a clear solution.

### Example 74 composition comprising compound 5 and SBE6.5-β-CD

6.42 mg of compound 5 was weighed and dissolved with 0.1M of hydrochloric acid to prepare 2 mg/ml of solution. 29.43 mg (about 3 folds of molar weight) of SBE6.5-β-CD was added into the resultant solution with vortexing. 0.1M of sodium hydroxide solution was dropwise added to such an extent that precipitates were just to generate and at the same time the precipitates can disappear. The solvent was removed by freeze-drying to obtain the desired composition 74. To 2 ml of water was added the resultant composition to obtain a clear solution.

### Example 75 composition comprising compound 6 and SBE6.5-β-CD

3.79 mg of compound 6 was weighed and dissolved with 0.1M of hydrochloric acid to prepare 2 mg/ml of solution. 31.96 mg (about 3 folds of molar weight) of SBE6.5-β-CD was added into the resultant solution with vortexing. 0.1M of sodium hydroxide solution was dropwise added to such an extent that precipitates were just to generate and at the same time the precipitates can disappear. The solvent was removed by freeze-drying to obtain the desired composition 75. To 2 ml of water was added the resultant composition to obtain a clear solution.

### Example 76 composition comprising compound 8 and SBE6.5-β-CD

2.98 mg of compound 8 was weighed and dissolved with 0.1M of hydrochloric acid to prepare 2 mg/ml of solution. 31.64 mg (about 3 folds of molar weight) of SBE6.5-β-CD was added into the resultant solution with vortexing. 0.1M of sodium hydroxide solution was dropwise added to such an extent that precipitates were just to generate and at the same time the precipitates can disappear. The solvent was removed by freeze-drying to obtain the desired composition 76. To 2 ml of water was added the resultant composition to obtain a clear solution.

### Example 77 composition comprising compound 9 and SBE6.5-β-CD

5.12 mg of compound 9 was weighed and dissolved with0.1M of hydrochloric acid to prepare 2 mg/ml of solution. 32.03 mg (about 3 folds of molar weight) of SBE6.5-β-CD was added into the resultant solution with vortexing. 0.1M of sodium hydroxide solution was dropwise added to such an extent that precipitates were just to generate and at the same time the precipitates can disappear. The solvent was removed by freeze-drying to obtain the desired composition 77. To 2 ml of water was added the resultant composition to obtain a clear solution.

### Example 78 composition comprising compound 10 and SBE6.5-β-CD

6.36 mg of compound 10 was weighed and dissolved with 0.1M of hydrochloric acid to prepare 2 mg/ml of solution. 31.00 mg (about 3 folds of molar weight) of SBE6.5-β-CD was added into the resultant solution with vortexing. 0.1M of sodium hydroxide solution was dropwise added to such an extent that precipitates were just to generate and at the same time the precipitates can disappear. The solvent was removed by freeze-drying to obtain the desired composition 78. To 2 ml of water was added the resultant composition to obtain a clear solution.

### Example 79 composition comprising compound 16 and SBE6.5-β-CD

3.21 mg of compound 16 was weighed and dissolved with0.1M of hydrochloric acid to prepare 2 mg/ml of solution. 30.45 mg (about 3 folds of molar weight) of SBE6.5-β-CD was added into the resultant solution with vortexing. 0.1M of sodium hydroxide solution was dropwise added to such an extent that precipitates were just to generate and at the same time the precipitates can disappear. The solvent was removed by freeze-drying to obtain the desired composition 79. To 2 ml of water was added the resultant composition to obtain a clear solution.

### Example 80 composition comprising compound 17 and SBE6.5-β-CD

5.45 mg of compound 17 was weighed and dissolved with 0.1M of hydrochloric acid to prepare 2 mg/ml of solution. 30.01 mg (about 3 folds of molar weight) of SBE6.5-β-CD was added into the resultant solution with vortexing. 0.1M of sodium hydroxide solution was dropwise added to such an extent that precipitates were just to generate and at the same time the precipitates can disappear. The solvent was removed by freeze-drying to obtain the desired composition 80. To 2 ml of water was added the resultant composition to obtain a clear solution.

### Example 81 composition comprising compound 1 and SBE6-β-CD

3.76 mg of compound 1 was weighed and dissolved with 0.1M of hydrochloric acid to prepare 2 mg/ml of solution. 35.04 mg (about 3 folds of molar weight) of SBE6-β-CD was added into the resultant solution with vortexing. 0.1M of sodium hydroxide solution was dropwise added to such an extent that precipitates were just to generate and at the same time the precipitates can disappear. The solvent was removed by freeze-drying to obtain the desired composition 81. To 2 ml of water was added the resultant composition to obtain a clear solution.

### Example 82 composition comprising compound 16 and HP-β-CD

9.16 mg of compound 16 was weighed and dissolved with 4.58 ml of 0.1M of hydrochloric acid. To 2 ml of the resultant solution was added 44 mg (about 3 folds of molar weight) of HP-β-CD with vortexing. 0.1M of sodium hydroxide solution was dropwise added to such an extent that precipitates were just to generate and at the same time the precipitates can disappear. The solvent was removed by freeze-drying to obtain the desired composition 82.

### Example 83 composition comprising compound 16 and SBE7-β-CD

9.16 mg of compound 16 was weighed and dissolved with 4.58 ml of 0.1M of hydrochloric acid. To 2 ml of the resultant solution was added 65 mg (about 3 folds of molar weight) of SBE7-β-CD with vortexing. 0.1M of sodium hydroxide solution was dropwise added to such an extent that the precipitates were just to generate and at the same time the precipitates can disappear. The solvent was removed by freeze-drying to obtain the desired composition 83.

### Example 84 composition comprising compound 17 and HP-β-CD

8.36 mg of compound 17 was weighed and dissolved with 4.18 ml of 0.1M of hydrochloric acid. To 2 ml of the resultant solution was added 42 mg (about 3 folds of molar weight) of HP-β-CD with vortexing. 0.1M of sodium hydroxide solution was dropwise added to such an extent that precipitates were just to generate and at the same time the precipitates can disappear. The solvent was removed by freeze-drying to obtain the desired composition 84.

### Example 85 composition comprising compound 17 and SBE7-β-CD

8.36 mg of compound 17 was weighed and dissolved with 4.18 ml of 0.1M of hydrochloric acid. To 2 ml of the resultant solution was added 63 mg (about 3 folds of molar weight) of SBE7-β-CD with vortexing. 0.1M of sodium hydroxide solution was dropwise added to such an extent that precipitates were just to generate and at the same time the precipitates can disappear. The solvent was removed by freeze-drying to obtain the desired composition 85.

After the compositions in Example 82 to Example 85 were redissolved in water, the control solutions used in determination of the solubility of compound 16 and compound 17 were used as control, respectively. The concentrations of the obtained solutions were determined by HPLC. The results were listed in Table 6.

**Table 6. Concentration of the redissolved solution of each composition in Example 82 to Example 85 (Unit: µg/ml)**

| Examples | Concentrations | Folds of Solubilization relative to compound 16 or 17 |
|---|---|---|
| Example 82 | 1260 | 72 |
| Example 83 | 1198 | 68 |
| Example 84 | 1508 | 225 |
| Example 85 | 1575 | 235 |

## Claims

1. A composition comprising a compound of formula (I), a pharmaceutically acceptable salt of a compound of formula (I), a solvate of a compound of formula (I), a solvate of a pharmaceutically acceptable salt of a compound of formula (I), or a tautomer of a compound of formula (I), and a cyclodextrin, a cyclodextrin derivative and/or a surfactant with solubilization, 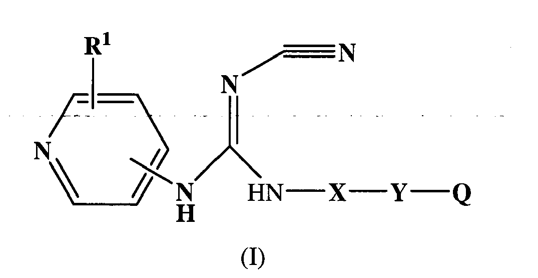 wherein
the connecting position with the pyridine ring is at the 3- or 4-position of the pyridine ring;
R¹ represents 0 to 4 following identical or different substituents: F, Cl, Br, NO₂, OH, COOH, CF₃, NR³R⁴, OR³, COOC₁₋₄ alkyl, C₁₋₄ alkyl or CN;
X represents C₄₋₂₀ alkylene;
Y represents a covalent bond, O, S, S(O), S(O)₂, NR³, -OC(O)-, -C(O)O-, -NHC(O)- or -C(O)NH-;
Q represents -C(O)OC₁₋₆ alkyl, a five- or six-membered aromatic heterocyclic ring containing 1 to 2 nitrogen atoms, a compound of formula (II) or a compound of formula (III), wherein the dashed lines in the compound of formula (II) and the compound of formula (III) represent the connecting position of chemical bonds, and R¹ in the compound of formula (III) is defined as R¹ in the compound of formula (I), 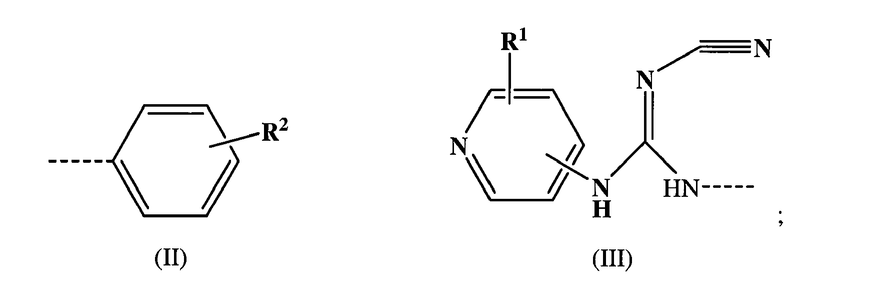
R² represents 0 to 5 following identical or different substituents: F, Cl, Br, NO₂ OH, COOH, CF₃, NR³R⁴, OR³, COOC₁₋₄ alkyl, C₁₋₄ alkyl, SC₁₋₄ alkyl, S(O)C₁₋₄ alkyl, S(O)₂C₁₋₄ alkyl or CN;
R³ and R⁴ represent H, C₁₋₄ alkyl, C(O)C₁₋₄ alkyl or S(O)₂C₁₋₄ alkyl, respectively.

2. A composition of claim 1, **characterized in that** the compound of formula (I) in the composition is selected from the following compounds or pharmaceutically acceptable salts thereof:
1) N-cyano-N'-(6-phenoxyhexyl)-N"-(3-pyridyl)guanidine,
2) N-cyano-N'-(6-(p-chlorophenoxy)hexyl)-N"-(3-pyridyl)guanidine,
3) N-cyano-N'-(6-(o-chlorophenoxy)hexyl)-N"-(3-pyridyl)guanidine,
4) N-cyano-N'-(6-(o-methoxyphenoxy)hexyl)-N"-(3-pyridyl)guanidine,
5) N-cyano-N'-(6-(o-nitrophenoxy)hexyl)-N"-(3-pyridyl)guanidine,
6) N-cyano-N'-(6-(m-chlorophenoxy)hexyl)-N"-(3-pyridyl)guanidine,
7) N-cyano-N'-(6-(m-methoxyphenoxy)hexyl)-N"-(3-pyridyl)guanidine,
8) N-cyano-N'-(6-(m-nitrophenoxy)hexyl)-N"-(3-pyridyl)guanidine,
9) N-cyano-N'-(6-(p-fluorophenoxy)hexyl)-N"-(3-pyridyl)guanidine,
10) N-cyano-N'-(6-(m-fluorophenoxy)hexyl)-N"-(3-pyridyl)guanidine,
11) N-cyano-N'-(6-(o-fluorophenoxy)hexyl)-N"-(3-pyridyl)guanidine,
12) N-cyano-N'-(6-(p-fluorophenoxy)hexyl)-N"-(4-pyridyl)guanidine,
13) N-cyano-N'-(6-(m-fluorophenoxy)hexyl)-N"-(4-pyridyl)guanidine,
14) N-cyano-N'-(6-(o-fluorophenoxy)hexyl)-N"-(4-pyridyl)guanidine,
15) N-cyano-N'-(6-(p-methoxyphenoxy)hexyl)-N"-(3-pyridyl)guanidine,
16) N-cyano-N'-(6-(p-nitrophenoxy)hexyl)-N"-(3-pyridyl)guanidine,
17) N-cyano-N'-(6-(p-nitrophenoxy)hexyl)-N"-(4-pyridyl)guanidine,
18) N-cyano-N'-(6-(p-methoxyphenoxy)hexyl)-N"-(4-pyridyl)guanidine,
19) N-cyano-N'-(6-(p-chlorophenoxy)hexyl)-N"-(4-pyridyl)guanidine,
20) N-cyano-N'-(6-(m-nitrophenoxy)hexyl)-N"-(4-pyridyl)guanidine,
21) N-cyano-N'-(6-(m-methoxyphenoxy)hexyl)-N"-(4-pyridyl)guanidine,
22) N-cyano-N'-(6-(m-chlorophenoxy)hexyl)-N"-(4-pyridyl)guanidine,
23) N-cyano-N'-(6-(o-nitrophenoxy)hexyl)-N"-(4-pyridyl)guanidine,
24) N-cyano-N'-(6-(o-methoxyphenoxy)hexyl)-N"-(4-pyridyl)guanidine,
25) N-cyano-N'-(6-(o-chlorophenoxy)hexyl)-N"-(4-pyridyl)guanidine,
26) N-cyano-N'-(6-phenoxyhexyl)-N"-(4-pyridyl)guanidine,
27) N-cyano-N'-(6-(2,4-dichlorophenoxy)hexyl)-N"-(4-pyridyl)guanidine,
28) N-cyano-N'-(6-(2,4,5-trichlorophenoxy)hexyl)-N"-(4-pyridyl)guanidine,
29) N-cyano-N'-(7-phenoxyheptyl)-N"-(3-pyridyl)guanidine,
30) N-cyano-N'-(7-phenoxyheptyl)-N"-(4-pyridyl)guanidine,
31) N-cyano-N'-(7-(p-fluorophenoxy)heptyl)-N"-(3-pyridyl)guanidine,
32) N-cyano-N'-(7-(o-fluorophenoxy)heptyl)-N"-(3-pyridyl)guanidine,
33) N-cyano-N'-(7-(m-fluorophenoxy)heptyl)-N"-(3-pyridyl)guanidine,
34) N-cyano-N'-(7-(p-fluorophenoxy)heptyl)-N"-(4-pyridyl)guanidine,
35) N-cyano-N'-(7-(o-fluorophenoxy)heptyl)-N"-(4-pyridyl)guanidine,
36) N-cyano-N'-(7-(m-fluorophenoxy)heptyl)-N"-(4-pyridyl)guanidine
37) N-cyano-N'-(7-(o-chlorophenoxy)heptyl)-N"-(4-pyridyl)guanidine,
38) N-cyano-N'-(7-(o-chlorophenoxy)heptyl)-N"-(3-pyridyl)guanidine,
39) N-cyano-N'-(7-(o-methoxyphenoxy)heptyl)-N"-(3-pyridyl)guanidine,
40) N-cyano-N'-(7-(o-methoxyphenoxy)heptyl)-N"-(4-pyridyl)guanidine,
41) N-cyano-N'-(7-(o-nitrophenoxy)heptyl)-N"-(4-pyridyl)guanidine,
42) N-cyano-N'-(7-(o-nitrophenoxy)heptyl)-N"-(3-pyridyl)guanidine,
43) N-cyano-N'-(7-(m-chlorophenoxy)heptyl)-N"-(3-pyridyl)guanidine,
44) N-cyano-N'-(7-(m-chlorophenoxy)heptyl)-N"-(4-pyridyl)guanidine,
45) N-cyano-N'-(7-(m-methoxyphenoxy)heptyl)-N"-(4-pyridyl)guanidine,
46) N-cyano-N'-(7-(m-methoxyphenoxy)heptyl)-N"-(3-pyridyl)guanidine,
47) N-cyano-N'-(7-(m-nitrophenoxy)heptyl)-N"-(3-pyridyl)guanidine,
48) N-cyano-N'-(7-(m-nitrophenoxy)heptyl)-N"-(4-pyridyl)guanidine,
49) N-cyano-N'-(7-(p-chlorophenoxy)heptyl)-N"-(4-pyridyl)guanidine,
50) N-cyano-N'-(7-(p-chlorophenoxy)heptyl)-N"-(3-pyridyl)guanidine,
51) N-cyano-N'-(7-(p-methoxyphenoxy)heptyl)-N"-(3-pyridyl)guanidine,
52) N-cyano-N'-(7-(p-methoxyphenoxy)heptyl)-N"-(4-pyridyl)guanidine,
53) N-cyano-N'-(7-(p-nitrophenoxy)heptyl)-N"-(4-pyridyl)guanidine,
54) N-cyano-N'-(7-(p-nitrophenoxy)heptyl)-N"-(3-pyridyl)guanidine,
55) N-cyano-N'-(5-(p-fluorophenoxy)pentyl)-N"-(3-pyridyl)guanidine,
56) N-cyano-N'-(5-(o-fluorophenoxy)pentyl)-N"-(3-pyridyl)guanidine,
57) N-cyano-N'-(5-(m-fluorophenoxy)pentyl)-N"-(3-pyridyl)guanidine,
58) N-cyano-N'-(5-(p-fluorophenoxy)pentyl)-N"-(4-pyridyl)guanidine,
59) N-cyano-N'-(5-(o-fluorophenoxy)pentyl)-N"-(4-pyridyl)guanidine,
60) N-cyano-N'-(5-(m-fluorophenoxy)pentyl)-N"-(4-pyridyl)guanidine
61) N-cyano-N'-(5-(p-chlorophenoxy)pentyl)-N"-(3-pyridyl)guanidine,
62) N-cyano-N'-(5-phenoxypentyl)-N"-(3-pyridyl)guanidine,
63) N-cyano-N'-(5-phenoxypentyl)-N"-(4-pyridyl)guanidine,
64) N-cyano-N'-(5-(p-chlorophenoxy)pentyl)-N"-(4-pyridyl)guanidine,
65) N-cyano-N'-(5-(p-methoxyphenoxy)pentyl)-N"-(3-pyridyl)guanidine,
66) N-cyano-N'-(5-(o-chlorophenoxy)pentyl)-N"-(3-pyridyl)guanidine,
67) N-cyano-N'-(5-(m-chlorophenoxy)pentyl)-N"-(3-pyridyl)guanidine,
68) N-cyano-N'-(5-(p-methoxyphenoxy)pentyl)-N"-(4-pyridyl)guanidine,
69) N-cyano-N'-(5-(o-chlorophenoxy)pentyl)-N"-(4-pyridyl)guanidine,
70) N-cyano-N'-(5-(m-chlorophenoxy)pentyl)-N"-(4-pyridyl)guanidine
71) N-cyano-N'-(8-(p-fluorophenoxy)octyl)-N"-(3-pyridyl)guanidine,
72) N-cyano-N'-(8-(o-fluorophenoxy)octyl)-N"-(3-pyridyl)guanidine,
73) N-cyano-N'-(8-(m-fluorophenoxy)octyl)-N"-(3-pyridyl)guanidine,
74) N-cyano-N'-(8-(p-fluorophenoxy)octyl)-N"-(4-pyridyl)guanidine,
75) N-cyano-N'-(8-(o-fluorophenoxy)octyl)-N"-(4-pyridyl)guanidine,
76) N-cyano-N'-(8-(m-fluorophenoxy)octyl)-N"-(4-pyridyl)guanidine.
77) N-cyano-N'-(8-(p-chlorophenoxy)octyl)-N"-(4-pyridyl)guanidine,
78) N-cyano-N'-(8-(p-chlorophenoxy)octyl)-N"-(3-pyridyl)guanidine,
79) N-cyano-N'-(8-phenoxyoctyl)-N"-(3-pyridyl)guanidine,
80) N-cyano-N'-(8-phenoxyoctyl)-N"-(4-pyridyl)guanidine,
81) N-cyano-N'-(10-phenoxydecyl)-N"-(4-pyridyl)guanidine,
82) N-cyano-N'-(10-phenoxydecyl)-N"-(3-pyridyl)guanidine,
83) N-cyano-N'-(10-(p-chlorophenoxy)decyl)-N"-(3-pyridyl)guanidine,
84) N-cyano-N'-(10-(o-chlorophenoxy)decyl)-N"-(3-pyridyl)guanidine,
85) N-cyano-N'-(10-(m-chlorophenoxy)decyl)-N"-(3-pyridyl)guanidine,
86) N-cyano-N'-(10-(p-fluorophenoxy)decyl)-N"-(3-pyridyl)guanidine,
87) N-cyano-N'-(10-(m-fluorophenoxy)decyl)-N"-(3-pyridyl)guanidine,
88) N-cyano-N'-(10-(o-fluorophenoxy)decyl)-N"-(3-pyridyl)guanidine,
89) N-cyano-N'-(10-(p-methoxyphenoxy)decyl)-N"-(3-pyridyl)guanidine,
90) N-cyano-N'-(10-(m-methoxyphenoxy)decyl)-N"-(3-pyridyl)guanidine,
91) N-cyano-N'-(10-(o-methoxyphenoxy)decyl)-N"-(3-pyridyl)guanidine,
92) N-cyano-N'-(10-(p-nitrophenoxy)decyl)-N"-(3-pyridyl)guanidine,
93) N-cyano-N'-(10-(o-nitrophenoxy)decyl)-N"-(3-pyridyl)guanidine,
94) N-cyano-N'-(10-(m-nitrophenoxy)decyl)-N"-(3-pyridyl)guanidine,
95) N-cyano-N'-(10-(p-chlorophenoxy)decyl)-N"-(4-pyridyl)guanidine,
96) N-cyano-N'-(10-(o-chlorophenoxy)decyl)-N"-(4-pyridyl)guanidine,
97) N-cyano-N'-(10-(m-chlorophenoxy)decyl)-N"-(4-pyridyl)guanidine,
98) N-cyano-N'-(10-(p-fluorophenoxy)decyl)-N"-(4-pyridyl)guanidine,
99) N-cyano-N'-(10-(m-fluorophenoxy)decyl)-N"-(4-pyridyl)guanidine,
100) N-cyano-N'-(10-(o-fluorophenoxy)decyl)-N"-(4-pyridyl)guanidine,
101) N-cyano-N'-(10-(p-methoxyphenoxy)decyl)-N"-(4-pyridyl)guanidine,
102) N-cyano-N'-(10-(m-methoxyphenoxy)decyl)-N"-(4-pyridyl)guanidine,
103) N-cyano-N'-(10-(o-methoxyphenoxy)decyl)-N"-(4-pyridyl)guanidine,
104) N-cyano-N'-(10-(p-nitrophenoxy)decyl)-N"-(4-pyridyl)guanidine,
105) N-cyano-N'-(10-(o-nitrophenoxy)decyl)-N"-(4-pyridyl)guanidine,
106) N-cyano-N'-(10-(m-nitrophenoxy)decyl)-N"-(4-pyridyl)guanidine,
107) N-cyano-N'-(12-phenoxydodecyl)-N"-(4-pyridyl)guanidine,
108) N-cyano-N'-(12-phenoxydodecyl)-N"-(3-pyridyl)guanidine,
109) N-cyano-N'-(12-(p-chlorophenoxy)dodecyl)-N"-(3-pyridyl)guanidine,
110) N-cyano-N'-(12-(o-chlorophenoxy)dodecyl)-N"-(3-pyridyl)guanidine,
111) N-cyano-N'-(12-(m-chlorophenoxy)dodecyl)-N"-(3-pyridyl)guanidine,
112) N-cyano-N'-(12-(p-fluorophenoxy)dodecyl)-N"-(3-pyridyl)guanidine,
113) N-cyano-N'-(12-(m-fluorophenoxy)dodecyl)-N"-(3-pyridyl)guanidine,
114) N-cyano-N'-(12-(o-fluorophenoxy)dodecyl)-N"-(3-pyridyl)guanidine,
115) N-cyano-N'-(12-(p-methoxyphenoxy)dodecyl)-N"-(3-pyridyl)guanidine,
116) N-cyano-N'-(12-(m-methoxyphenoxy)dodecyl)-N"-(3-pyridyl)guanidine,
117) N-cyano-N'-(12-(o-methoxyphenoxy)dodecyl)-N"-(3-pyridyl)guanidine,
118) N-cyano-N'-(12-(p-nitrophenoxy)dodecyl)-N"-(3-pyridyl)guanidine,
119) N-cyano-N'-(12-(o-nitrophenoxy)dodecyl)-N"-(3-pyridyl)guanidine,
120) N-cyano-N'-(12-(m-nitrophenoxy)dodecyl)-N"-(3-pyridyl)guanidine,
121) N-cyano-N'-(12-(p-chlorophenoxy)dodecyl)-N"-(4-pyridyl)guanidine,
122) N-cyano-N'-(12-(o-chlorophenoxy)dodecyl)-N"-(4-pyridyl)guanidine,
123) N-cyano-N'-(12-(m-chlorophenoxy)dodecyl)-N"-(4-pyridyl)guanidine,
124) N-cyano-N'-(12-(p-fluorophenoxy)dodecyl)-N"-(4-pyridyl)guanidine,
125) N-cyano-N'-(12-(m-fluorophenoxy)dodecyl)-N"-(4-pyridyl)guanidine,
126) N-cyano-N'-(12-(o-fluorophenoxy)dodecyl)-N"-(4-pyridyl)guanidine,
127) N-cyano-N'-(12-(p-methoxyphenoxy)dodecyl)-N"-(4-pyridyl)guanidine,
128) N-cyano-N'-(12-(m-methoxyphenoxy)dodecyl)-N"-(4-pyridyl)guanidine,
129) N-cyano-N'-(12-(o-methoxyphenoxy)dodecyl)-N"-(4-pyridyl)guanidine,
130) N-cyano-N'-(12-(p-nitrophenoxy)dodecyl)-N"-(4-pyridyl)guanidine,
131) N-cyano-N'-(12-(o-nitrophenoxy)dodecyl)-N"-(4-pyridyl)guanidine,
132) N-cyano-N'-(12-(m-nitrophenoxy)dodecyl)-N"-(4-pyridyl)guanidine,
133) 1,12-bis(N'-cyano-N"-4-pyridylguanidine)dodecane,
134) N-(12-(tert-butoxycarbonylamino)dodecyl)-N'-cyano-N"-(4-pyridyl)guanidine,
135) N-(6-((4-chlorobenzoic acid)ester)hexyl)-N'-cyano-N"-(4-pyridyl)guanidine,
136) N-cyano-N'-(6-(2-chloro-4-fluorophenyl)oxo)hexyl)-N"-(4-pyridyl)guanidine,
137) N-cyano-N'-(6-(o-bromophenoxy)hexyl)-N"-(4-pyridyl)guanidine,
138) N-cyano-N'-(6-((2,6-dichlorophenyl)oxo)hexyl)-N"-(4-pyridyl)guanidine,
139) N-cyano-N'-(6-((2,4,6-trichlorophenyl)oxo)hexyl)-N"-(4-pyridyl)guanidine,
140) N-cyano-N'-(6-(o-trifluoromethoxyphenyl)oxo)hexyl)-N"-(4-pyridyl)guanidine,
141) N-cyano-N'-(5-(o-chlorophenoxy)pentyl)-N"-(4-pyridyl)guanidine,
142) N-cyano-N'-(7-(o-chlorophenoxy)heptyl)-N"-(4-pyridyl)guanidine,
143) N-cyano-N'-(5-(p-chlorophenoxy)pentyl)-N"-(4-pyridyl)guanidine,
144) N-cyano-N'-(8-((o-methoxyphenyl)oxo)octyl)-N"-(4-pyridyl)guanidine,
145) N-cyano-N'-(5-((2,4-dichlorophenyl)oxo)pentyl)-N"-(4-pyridyl)guanidine, or
146) N-cyano-N'-(5-((2,4,6-trichlorophenyl)oxo)pentyl)-N"-(4-pyridyl)guanidine.

3. A composition of claim 1, **characterized in that** the compound of formula (I) in the composition is selected from the following compounds or pharmaceutically acceptable salts thereof:
Compound 1,
N-cyano-N'-(6-(p-chlorophenoxy)hexyl)-N"-(4-pyridyl)guanidine,
Compound 2,
N-cyano-N'-(6-(o-chlorophenoxy)hexyl)-N"-(4-pyridyl)guanidine,
Compound 3,
N-cyano-N'-(6-(o-methoxyphenoxy)hexyl)-N"-(4-pyridyl)guanidine,
Compound 4, 1,12-bis(N'-cyano-N"-4-pyridylguanidine)dodecane,
Compound 5,
N-(12-(tert-butoxycarbonylamino)dodecyl)-N'-cyano-N"-(4-pyridyl)guanidine,
Compound 6,
N-(6-((4-chlorobenzoicacid)ester)hexyl)-N'-cyano-N"-(4-pyridyl)guanidine,
Compound 7,
N-cyano-N'-(6-(2-chloro-4-fluorophenyl)oxo)hexyl)-N"-(4-pyridyl)guanidine,
Compound 8,
N-cyano-N'-(6-(o-bromophenoxy)hexyl)-N"-(4-pyridyl)guanidine,
Compound 9,
N-cyano-N'-(6-((2,6-dichlorophenyl)oxo)hexyl)-N"-(4-pyridyl)guanidine,
Compound 10,
N-cyano-N'-(6-(o-trifluoromethoxyphenyl)oxo)hexyl)-N"-(4-pyridyl)guanidine,
Compound 11,
N-cyano-N'-(5-(o-chlorophenoxy)pentyl)-N"-(4-pyridyl)guanidine,
Compound 12,
N-cyano-N'-(7-(o-chlorophenoxy)heptyl)-N"-(4-pyridyl)guanidine,
Compound 13,
N-cyano-N'-(5-(p-chlorophenoxy)pentyl)-N"-(4-pyridyl)guanidine,
Compound 14,
N-cyano-N'-(8-((o-methoxyphenyl)oxo)octyl)-N"-(4-pyridyl)guanidine,
Compound 15,
N-cyano-N'-(5-((2,4-dichlorophenyl)oxo)pentyl)-N"-(4-pyridyl)guanidine,
Compound 16,
N-cyano-N'-(5-((2,4,6-trichlorophenyl)oxo)pentyl)-N"-(4-pyridyl)guanidine, or
Compound 17,
N-cyano-N'-(6-((2,4,6-trichlorophenyl)oxo)hexyl)-N"-(4-pyridyl)guanidine.

4. A composition of any one of claims 1 to 3, **characterized in that** the cyclodextrin or the cyclodextrin derivative in the composition is selected from one or more than one of the following: α-cyclodextrin, β-cyclodextrin, γ-cyclodextrin, hydroxyethyl-β-CD, hydroxypropyl-β-CD, dihydroxypropyl-β-CD, sulfobutylether-β-CD, methyl-β-CD, dimethyl-β-CD, randomly dimethylated-β-CD, randomly methylated-β-CD, carboxy methyl-β-CD, carboxy methylethyl-β-CD, diethyl-β-CD, tris-O-methyl-β-CD, tris-O-ethyl-β-CD, tris-O-butyryl-β-CD, tris-O-valeryl-β-CD, bis-O-caproyl-β-CD, glucosyl-β-CD or maltosyl-β-CD, maltotriosyl-β-CD, succinyl-dimethyl-β-CD, maleyl-dimethyl-β-CD, sulfopropyl-β-CD and 6-carboxymethyl-β-CD (SBE-β-CD).

5. A composition of any one of claims 1 to 3, **characterized in that** the cyclodextrin or the cyclodextrin derivative in the composition is selected from the group consisting of β-cyclodextrin, hydroxyethyl-β-CD, hydroxypropyl-β-CD, dihydroxypropyl-β-CD, sulfobutylether-β-CD, carboxy methyl-β-CD, carboxy methylethyl-β-CD, glucosyl-β-CD or maltosyl-β-CD, maltotriosyl-β-CD and sulfopropyl-β-CD.

6. A composition of any one of claims 1 to 3, **characterized in that** the surfactant with solubilization in the composition is selected from one or more than one of the following: Tween-20, Tween-80, Tween-40, polyethylene glycol, polyoxyethylene castor oil, polyoxyethylene hydrogenated castor oil, polyvinylpyrrolidone, poloxamer, lecithin, soy lecithin, cephalin, phosphatidic acid, dipalmitoylphosphatidylcholine, hydrophosphatidylethanolamine or phosphatidylserine, cholesterol, diamino cholesterol, soy sterylacylglucoside, soysterol and ergosterol.

7. A composition of any one of claims 1 to 3, **characterized in that** the composition is selected from the group consisting of:
1) a composition comprising
N-cyano-N'-(6-(p-chlorophenoxy)hexyl)-N"-4-pyridyl guanidine or a pharmaceutically acceptable salt thereof and hydroxypropyl-β-CD,
2) a composition comprising
N-cyano-N'-(6-(p-chlorophenoxy)hexyl)-N"-4-pyridyl guanidine or a pharmaceutically acceptable salt thereof and sulfobutylether-β-CD,
3) a composition comprising
N-cyano-N'-(6-(o-chlorophenoxy)hexyl)-N"-4-pyridyl guanidine or a pharmaceutically acceptable salt thereof and hydroxypropyl-β-CD,
4) a composition comprising
N-cyano-N'-(6-(o-chlorophenoxy)hexyl)-N"-4-pyridyl guanidine or a pharmaceutically acceptable salt thereof and sulfobutylether-β-CD,
5) a composition comprising
N-cyano-N'-(6-(o-methoxyphenoxy)hexyl)-N"-4-pyridyl guanidine or a pharmaceutically acceptable salt thereof and hydroxypropyl-β-CD,
6) a composition comprising
N-cyano-N'-(6-(o-methoxyphenoxy)hexyl)-N"-4-pyridyl guanidine or a pharmaceutically acceptable salt thereof and sulfobutylether-β-CD,
7) a composition comprising
1,12-bis(N'-cyano-N"-4-pyridylguanidine)dodecane or a pharmaceutically acceptable salt thereof and hydroxypropyl-β-CD,
8) a composition comprising
1,12-bis(N'-cyano-N"-4-pyridylguanidine)dodecane or a pharmaceutically acceptable salt thereof and sulfobutylether-β-CD,
9) a composition comprising
N-(12-(tert-butoxycarbonylamino)dodecyl)-N'-cyano-N"-(4-pyridyl)guanidine or a pharmaceutically acceptable salt thereof and hydroxypropyl-β-CD,
10) a composition comprising
N-(12-(tert-butoxycarbonylamino)dodecyl)-N'-cyano-N"-(4-pyridyl)guanidine or a pharmaceutically acceptable salt thereof and sulfobutylether-β-CD,
11) a composition comprising N-(6-((4-chlorobenzoic acid)ester)hexyl)-N'-cyano-N"-(4-pyridyl)guanidine or a pharmaceutically acceptable salt thereof and hydroxypropyl-β-CD,
12) a composition comprising N-(6-((4-chlorobenzoic acid)ester)hexyl)-N'-cyano-N"-(4-pyridyl)guanidine or a pharmaceutically acceptable salt thereof and sulfobutylether-β-CD,
13) a composition comprising
N-cyano-N'-(6-(2-chloro-4-fluorophenyl)oxo)hexyl)-N"-(4-pyridyl)guanidine or a pharmaceutically acceptable salt thereof and hydroxypropyl-β-CD,
14) a composition comprising
N-cyano-N'-(6-(2-chloro-4-fluorophenyl)oxo)hexyl)-N"-(4-pyridyl)guanidine or a pharmaceutically acceptable salt thereof and sulfobutylether-β-CD,
15) a composition comprising
N-cyano-N'-(6-(o-bromophenoxy)hexyl)-N"-(4-pyridyl)guanidine or a pharmaceutically acceptable salt thereof and hydroxypropyl-β-CD,
16) a composition comprising
N-cyano-N'-(6-(o-bromophenoxy)hexyl)-N"-(4-pyridyl)guanidine or salt and sulfobutylether-β-CD,
17) a composition comprising
N-cyano-N'-(6-((2,6-dichlorophenyl)oxo)hexyl)-N"-(4-pyridyl)guanidine or a pharmaceutically acceptable salt thereof and hydroxypropyl-β-CD,
18) a composition comprising
N-cyano-N'-(6-((2,6-dichlorophenyl)oxo)hexyl)-N"-(4-pyridyl)guanidine or a pharmaceutically acceptable salt thereof and sulfobutylether-β-CD,
19) a composition comprising
N-cyano-N'-(6-(o-trifluoromethoxyphenyl)oxo)hexyl)-N"-(4-pyridyl)guanidine or a pharmaceutically acceptable salt thereof and hydroxypropyl-β-CD,
20) a composition comprising
N-cyano-N'-(6-(o-trifluoromethoxyphenyl)oxo)hexyl)-N"-(4-pyridyl)guanidine or a pharmaceutically acceptable salt thereof and sulfobutylether-β-CD,
21) a composition comprising
N-cyano-N'-(5-(o-chlorophenoxy)pentyl)-N"-(4-pyridyl)guanidine or a pharmaceutically acceptable salt thereof and hydroxypropyl-β-CD,
22) a composition comprising
N-cyano-N'-(5-(o-chlorophenoxy)pentyl)-N"-(4-pyridyl)guanidine or a pharmaceutically acceptable salt thereof and sulfobutylether-β-CD,
23) a composition comprising
N-cyano-N'-(7-(o-chlorophenoxy)heptyl)-N"-(4-pyridyl)guanidine or a pharmaceutically acceptable salt thereof and hydroxypropyl-β-CD,
24) a composition comprising
N-cyano-N'-(7-(o-chlorophenoxy)heptyl)-N"-(4-pyridyl)guanidine or a pharmaceutically acceptable salt thereof and sulfobutylether-β-CD,
25) a composition comprising
N-cyano-N'-(5-(p-chlorophenoxy)pentyl)-N"-(4-pyridyl)guanidine or a pharmaceutically acceptable salt thereof and hydroxypropyl-β-CD,
26) a composition comprising
N-cyano-N'-(5-(p-chlorophenoxy)pentyl)-N"-(4-pyridyl)guanidine or a pharmaceutically acceptable salt thereof and sulfobutylether-β-CD,
27) a composition comprising
N-cyano-N'-(8-((o-methoxyphenyl)oxo)octyl)-N"-(4-pyridyl)guanidine or a pharmaceutically acceptable salt thereof and hydroxypropyl-β-CD,
28) a composition comprising
N-cyano-N'-(8-((o-methoxyphenyl)oxo)octyl)-N"-(4-pyridyl)guanidine or a pharmaceutically acceptable salt thereof and sulfobutylether-β-CD,
29) a composition comprising
N-cyano-N'-(5-((2,4-dichlorophenyl)oxo)pentyl)-N"-(4-pyridyl)guanidine or a pharmaceutically acceptable salt thereof and hydroxypropyl-β-CD,
30) a composition comprising
N-cyano-N'-(5-((2,4-dichlorophenyl)oxo)pentyl)-N"-(4-pyridyl)guanidine or a pharmaceutically acceptable salt thereof and sulfobutylether-β-CD,
31) a composition comprising
N-cyano-N'-(6-((2,4,6-trichlorophenyl)oxo)hexyl)-N"-(4-pyridyl)guanidine or a pharmaceutically acceptable salt thereof and sulfobutylether-β-CD,
32) a composition comprising
N-cyano-N'-(6-((2,4,6-trichlorophenyl)oxo)hexyl)-N"-(4-pyridyl)guanidine or a pharmaceutically acceptable salt thereof and hydroxypropyl-β-CD,
33) a composition comprising
N-cyano-N'-(5-((2,4,6-trichlorophenyl)oxo)pentyl)-N"-(4-pyridyl)guanidine or a pharmaceutically acceptable salt thereof and sulfobutylether-β-CD, and
34) a composition comprising
N-cyano-N'-(5-((2,4,6-trichlorophenyl)oxo)pentyl)-N"-(4-pyridyl)guanidine or a pharmaceutically acceptable salt thereof and hydroxypropyl-β-CD.

8. A composition of any one of claims 1 to 7, **characterized in that** the pharmaceutically acceptable salt is formed with a compound of formula (I) and one or more than one of the following: hydrochloric acid, hydrobromic acid, hydroiodic acid, sulfuric acid, nitric acid, acetic acid, phosphoric acid, lactic acid, maleic acid, phthalic acid, citric acid, propionic acid, benzoic acid, glutaric acid, gluconic acid, methanesulfonic acid, salicylic acid, succinic acid, tartaric acid, toluenesulfonic acid, aminosulfonic acid, fumaric acid, formic acid, malic acid, ascorbic acid, malonic acid, oxalic acid, hydroxyacetic acid and benzenesulfonic acid.

9. A composition of any one of claims 1 to 7, **characterized in that** the composition is prepared as a medicament for treating a disease induced by abnormal cell proliferation.

10. A composition of claim 9, wherein the disease induced by abnormal cell proliferation is cancer.

11. A-composition of claim 9 or 10, wherein the disease induced by abnormal cell proliferation is leukocythemia, acute myelocytic leukemia, chronic myelocytic leukemia, chronic lymphocytic leukemia, myelodysplasia, multiple myeloma, Hodgkin's disease or non-Hodgkin's lymphoma, small cell or non-small cell lung cancer, gastric cancer, bowel cancer, colon cancer, rectal cancer, prostate cancer, ovarian cancer, breast cancer, brain cancer, head and neck cancer, urethral carcinoma, renal carcinoma, bladder cancer, melanoma, liver cancer, uterine cancer or pancreatic cancer.

12. A process for preparing a composition of any one of claims 1 to 7, comprising mixing a compound of formula (I) or a solution comprising a compound of formula (I) with a cyclodextrin, a cyclodextrin derivative and/or a surfactant with solubilization or a solution comprising a cyclodextrin, a cyclodextrin derivative and/or a surfactant with solubilization, and removing the solvent.

13. A composition of any one of claims 1 to 7, **characterized in that** the composition can be prepared as a pharmaceutical preparation to be applied via oral administration, buccal administration, intravenous administration, intraperitoneal injection, subcutaneous injection, intramuscular injection, nasal drops, eye drops, inhalation, anal administration, vaginal administration, epidermal administration and the like.

14. A composition of any one of claims 1 to 7, **characterized in that** the molar ratio of the compound of formula (I) to the cyclodextrin, the cyclodextrin derivative or the surfactant with solubilization in the composition is in the range of from 1:1000 to 200:1.

15. A composition of any one of claims 1 to 7, **characterized in that** the unit dosage of the compound of formula (I) in the composition is in the range of from 0.1 mg to 5 g.

16. A composition of any one of claims 1 to 7, **characterized in that** the composition can be used in combination with other antitumor compounds.

17. A composition of claim 16, wherein the other antitumor compounds are selected from one or more than one of the following: paclitaxel, fluorouracil, etoposide, cyclophosphamide, cisplatin, carboplatin, vincristine, gemcitabine, vinorelbine, chlorambucil, adriamycin, melphalan,-seocalcitol and epirubicin.
